# EUROPEAN PATENT APPLICATION

(11) **EP 1 669 026 A1**
(43) Date of publication of application: **14.06.2006**
(21) Application number: 04791955.0
(22) Date of filing: 01.10.2004
(51) Int. Cl.: A61B 5/07, A61B 1/04

(54) **BODY INSIDE OBSERVATION DEVICE**

(30) Priority: 01.10.2003 JP 2003343064; 22.10.2003 JP 2003361782
(71) Applicant: Olympus Corporation, Tokyo 151-0072 (JP)
(72) Inventor: KAWANO, Hironao, Hachioji-shi, Tokyo 1920032 (JP); UCHIYAMA, Akio, Yokohama-shi, Kanagawa 2220003 (JP); TAKIZAWA, Hironobu, Hachioji-shi, Tokyo 1930844 (JP); KIKUCHI, Akira, Yokohama-shi, Kanagawa 2260027 (JP)
(74) Representative: von Hellfeld, Axel
(86) International application number: PCT/JP2004/014488
(87) International publication number: WO 2005/032370

(57) **Abstract**

This in vivo observation device is equipped with a capsular casing that is taken orally into a living body, and an observation device provided within said casing which observes the inside of the living body through an observation wall surface of said casing; and, is provided with a contact auxiliary device that causes the observation wall surface to closely contact to body tissue during observation.

## Description

### TECHNICAL FIELD

The present invention relates to an in vivo observation device that is used to observe affected areas such as superficial diseases and so forth that have occurred in the digestive tract and other locations.

The present application claims priority from Japanese Patent Application No. 2003-343064 filed on October 1, 2003 and from Japanese Patent Application No. 2003-361782 filed on October 22, 2003, the contents of which are incorporated herein by reference.

### BACKGROUND ART

Inflammatory bowel diseases (IBD) such as ulcerative colitis and Crohn' s disease are diseases of the digestive tract for which their cause is still unknown, and it is currently imperative to establish various treatments and preventive methods. The symptoms of inflammatory bowel diseases consist of prolonged diarrhea, rectal bleeding and bloody stools lasting for a long period of time, and although they are rarely completely cured, these diseases are characterized nearly always by repeated recovery and relapse of colitis lesions extending for a long period of time. At present, treatment is dependent on long-term drug administration.

In addition, although the presence of bloody stools, endoscopic examinations or X-ray examinations are typically performed for diagnosing inflammatory bowel diseases, diagnostic methods by endoscopic examination are used preferably since they allow direct confirmation of images of the digestive tract. Since ulcerative colitis in particular, which is a kind of inflammatory bowel disease, is associated with an increased risk of cancer after a fixed period of time has elapsed following onset of the diseases (e.g., 7 years), progression of symptoms and so forth is confirmed by regular endoscopic examinations performed once a year.

On the other hand, capsular medical devices that are taken orally into the body are also known as devices that allow a patient's health to be examined easily. Various types of these capsular medical devices are available, including those that randomly take photographs of various locations in the body, those that collect samples from within the body, and those that release medication. As one example of such a device, a capsular bioinformation examination device is known that is capable of detecting biological information such as image information and so forth within the body (refer to, for example, Japanese Unexamined Patent Application, First Publication No. 11-225996 (paragraphs 0007-0030, Figs. 1 through 3)).

This capsular bioinformation examination device is provided with a casing having an optical output port for outputting illumination light within the body, an imaging port for capturing images within the body, and a bioinformation sensor that detects temperature within the body and so forth. In addition, the casing contains a battery for supplying power to each unit, a white LED for illuminating the body through the optical output port, a CCD for capturing images of the body through the imaging port, a control circuit for their control, and memory for storing bioinformation obtained from each unit. In addition, the white LED also serves as a transmission unit for transmitting bioinformation stored in memory to the outside.

In addition, there is a method for evaluating the presence or concentration of biological substances or chemical substances in blood vessels that uses a detection device that inserts a support into the body, immobilizes a reactant that demonstrates an optical change when reacting with a specific substance in that support, and detects that optical change (e. g. , WO 01/53792A2).

In addition, another example of a capsular medical device is known in which an ingestible capsule is provided with a camera device and optics, a video output inside the body is transmitted from the camera device by a transmitter, and then received with a receiver outside the body (e.g., USP 5,604,531).

In the case of performing examinations using this capsular bioinformation examination device, the patient swallows the capsular bioinformation examination device after the power switch has been turned on. After having been taken orally, the capsular bioinformation examination device captures images of each part of the body with the CCD by illuminating the inside of the body with the white LED while moving through organs within the body. This captured information is then stored in memory. In addition, information that has been obtained by bioinformation sensors is similarly stored in memory. After having detected bioinformation on various parts of the body, the capsular bioinformation examination device is excreted and recovered, after which the information stored in memory by means of the white LED is removed, analyzed and examined.

However, although it is necessary to regularly perform endoscopic examinations as previously described for inflammatory bowel diseases and for ulcerative colitis in particular, typically in the case of performing endoscopic examinations, it is necessary to flush the intestines until they are clean by excreting stool and food residue present in the intestines by drinking a laxative and so forth as a preliminary preparation for the examination in order to obtain accurate and clear images. However, since mucosa that protects intestinal tissue also ends up being flushed out in addition to stool and food residue during this flushing of the intestines, in the case of ulcerative colitis, body tissue is susceptible to reacting with excess sensitivity as a result of the loss of this mucosa, thereby resulting in the possibility of further exacerbation of symptoms. Consequently, since endoscopic examinations are frequently performed without flushing the intestines in cases of ulcerative colitis, this resulted in the problem of being unable to obtain clear images from inside the intestines.

In addition, even if a capsular medical device such as the capsular bioinformation examination device described in the aforementioned patent document 1 is used instead of an endoscope, there is still the possibility of being unable to obtain clear images inside the intestines.

In consideration of these circumstances, the object of the present invention is to provide an in vivo observation device that enables observation of an affected area in the digestive tract (and particularly the intestines) without flushing the intestines.

### DISCLOSURE OF THE INVENTION

This invention provides the following means to achieve the aforementioned objects.

The first invention provides an in vivo observation device equipped with: a capsular casing that is taken orally into a living body, and an observation device provided within said casing which observes the inside of the living body through an observation wall surface of said casing; and, is provided with a contact auxiliary device that causes the observation wall surface to closely contact to body tissue during observation.

The second invention is the in vivo observation device of the first invention wherein, the contact auxiliary device is provided within the casing.

The third invention is the in vivo observation device of the first invention wherein, the contact auxiliary device is an external contact auxiliary device provided with an action generating section that generates an operation in the casing from outside the living body.

The fourth invention is the in vivo observation device of the first invention wherein, the contact auxiliary device is composed of an external contact auxiliary device provided with an action generating section that generates an action in the casing from outside the living body, and a reacting section that receives the operation generated by the action generating section provided in the casing.

Furthermore, in the case the contact auxiliary device causes the in vivo observation device to be closely contacted to body tissue by applying pressure from outside the living body, the procedure can be simplified without requiring special tools if a physician and so forth applies pressure by hand.

According to this in vivo observation device, since a contact auxiliary device is provided that causes the observation wall to closely adhere to body tissue during observation, if the observation window is observed while closely adhered to the body tissue, impaired field of view caused by body fluids and other foreign objects present in the living body can be eliminated. Thus, an in vivo observation device for observing the inside of the body allows accurate observation inside the body by ensuring a satisfactory observation field.

The fifth invention is the in vivo observation device of the second invention wherein, the contact auxiliary device is equipped with a fluid transfer device installed within the casing which aspirates fluids such as body fluids and gases within the body and causes them to flow from the front of the observation wall surface to the rear, and causes the body tissue to be closely contacted to the observation wall surface as the fluids are aspirated by the fluid transfer device.

According to this type of in vivo observation device, since fluids within the body are aspirated from the front of the observation wall surface and allowed to flow to the rear by operating the fluid transfer device, body tissue is closely adhered to the observation wall surface by generating negative pressure within a lumen or organ in front of the observation wall surface.

The sixth invention is the in vivo observation device of the fifth invention wherein, the casing is provided with a cylindrical member that protrudes from the observation wall surface towards the direction of observation. As a result, the living body tissue desired to be observed can be efficiently and reliably aspirated and closely adhered to the observation wall surface.

The seventh invention is the in vivo observation device of the sixth invention wherein, the cylindrical member is removable. As a result, a cylindrical member of the optimum shape can be selected and used according to the observation conditions.

The eighth invention is the in vivo observation device of the fifth invention wherein, the observation wall surface is provided on the side portion of the casing. As a result, body tissue to the side can be observed easily. In addition, since body tissue observed by the in vivo observation device can be held in position, accurate observations can be made.

The ninth invention is the in vivo observation device of the eighth invention wherein, a foreign object removal device that protrudes from the outer periphery of the casing is provided around the observation wall surface. As a result, foreign objects can be prevented from entering the observation range.

The tenth invention is the in vivo observation device of the fifth invention wherein, an outer diameter expansion device is provided on the side of the casing. As a result, since the outer diameter expansion device blocks the lumen so as to divide the front and back of aspiration even in organs having a large lumen, body fluids and other fluids can be aspirated easily and efficiently.

The eleventh invention is the in vivo observation device of the second invention that is provided with a capsular casing that is taken orally into the living body, an observation device provided within said casing which observes the inside of the body, an optically transparent balloon provided on the casing so as to cover the periphery of the observation device and which can be expanded so as to closely contact to the living body when moving inside the living body, and an expansion device that expands the balloon by supplying a fluid inside the balloon;
wherein, the observation device observes the inside of the living body through the balloon.

In the in vivo internal observation device as claimed in this invention, when the observation device is taken orally and has reached, for example, the intestine by moving through the digestive tract, the expansion device supplies fluid to the balloon to expand the balloon causing the balloon to closely contact to the living body. Furthermore, the expansion device may be set to operate at its own discretion, or it may be set so as to operate after having received a signal from outside the living body. At this time, even there is body fluid or food residue between the outside of the balloon and the living body, since the balloon is expanded while pushing away any body fluid, food residue or other foreign objects, it closely contact to body tissue after having removed said foreign objects. When subsequently moving through the digestive tract, it similarly moves while maintaining a state in which foreign objects have been removed from between the balloon and body tissue. As a result, the observation device allows observation of the living body in the optimum state without being affected by foreign objects.

In this manner, since body tissue can be observed through a balloon by expanding the balloon causing it to closely adhered to body tissue, status of the living body can be reliably observed without having to flush out the intestines. In the case of inflammatory bowel diseases in particular, the status of the digestive tract including any affected areas can be reliably observed while preventing exacerbation of symptoms caused by flushing the intestines.

The twelfth invention is the in vivo observation device of the eleventh invention wherein, the expansion device is provided with an acquisition section that acquires body fluid inside the living body, and an expansion section that expands the balloon based on the liquid content of the living body fluid acquired with the acquisition section.

In the in vivo observation device as claimed in this invention, the acquisition section moves through the living body while acquiring body fluid. In addition, the expansion section expands the balloon in the case the moisture content of body fluid acquired with the acquisition section is equal to or greater than, for example, a preset specified amount. In this manner, the balloon can be expanded at a desired location within the living body by adjusting the moisture content of body fluid.

The thirteenth invention is the in vivo observation device of the eleventh invention wherein, a duct that connects the outside and inside of the balloon is provided in the casing, and the expansion device is provided with a pump that expands the balloon by supplying fluid from the outside to the inside of the balloon through the duct or contracts the balloon by discharging fluid to the outside from inside the balloon, and a control section that controls the operation of said pump.

In the in vivo observation device as claimed in this invention, the balloon is expanded supplying fluid from outside the casing to inside the balloon or the balloon is contracted by discharging fluid to outside the casing from inside the balloon as a result of the control section operating the pump. In this manner, since the balloon can be expanded by acquiring fluid from outside the living body, it is not necessary to provide a fluid inside the living body in advance. Thus, the constitution can be simplified and the size of the device can be reduced.

The fourteenth invention is the in vivo observation device of the eleventh invention wherein, another balloon is provided in the casing, ducts that respectively communicate with the insides of the balloon and the other balloon are provided within the casing, and the expansion device is provided with a pump that mutually supplies the fluid to both balloons through the ducts, and a control section that controls the operation of said pump.

In the in vivo observation device as claimed in this invention, the pump is operated by the control section to expand the first balloon by supplying fluid from the other balloon to the inside of said first balloon or contract the first balloon by supplying fluid from the inside of the first balloon to the other balloon. In this manner, since balloon expansion and contraction is carried out by transferring fluid between both balloons by operating the pump, it is not necessary to replace the fluid. Thus, the entry of foreign objects into the living body can be prevented, and the field of view of the observation device can be maintained in the optimum state.

The fifteenth invention is the in vivo observation device of the eleventh invention wherein, the balloon is provided with a reversing prevention device that has projections on the outer surface which protrude towards the rear with respect to the direction of travel.

In the in vivo observation device as claimed in this invention, the reversing prevention device also contacts the digestive tract or other part of the living body when the balloon is moved through while body while closely adhering thereto. At this time, since projections protrude towards the rear with respect to the direction of travel, in the case the balloon reverses direction within the digestive tract, the projections engage with the living body to prevent it from reversing. Thus, the balloon can be reliably moved in the predetermined direction of travel thereby allowing suitable observation.

The sixteenth invention is the in vivo observation device of the eleventh invention wherein, the balloon has an electrode on its outer surface, and an electrical power supply section is provided within the casing that supplies electrical power to the electrode.

In the in vivo observation device as claimed in this invention, current is made to flow within the living body as a result of the electrical power supply section supplying electrical power to the electrode when the balloon is moved through the living body while closely adhering thereto. The living body contracts when stimulated by the electrical current from the electrode, causing it to press against the balloon. As a result, since the balloon is subjected to external force from the living body, propulsion in the direction of travel is promoted or it is fed in towards the rear with respect to the direction of travel. Thus, in the case of desiring to reach a distal affected area or when desiring to make observations for a longer period of time, movement can be adjusted both easily and reliably by electrical stimulation.

The seventeenth invention is the in vivo observation device of the eleventh invention wherein, the fluid is an optically transparent liquid drug, micropores are provided in the balloon that allow the drug to be discharged outside the balloon when the balloon has been expanded at a pressure equal to or greater than a predetermined value, and the expansion device has a pressure raising device that raises the pressure inside the balloon to the pressure equal to or greater than the predetermined value.

In the in vivo observation device as claimed in this invention, a pressure raising device raises the pressure inside the balloon to a predetermined value or greater when the drug is released as a result of observation by the observation device or when a signal from outside the living body has been received. In addition, the pressure raising device is able to interrupt the release of drug by lowering the pressure inside the balloon. In this manner, a drug can be reliably administered at the desired location of an affected area by controlling the pressure inside the balloon with the pressure raising device. Since the balloon and affected area are in a closely adhered state free of body fluids and other foreign objects in particular, the drug can be administered directly to the affected area. In addition, since the fluid that expands the balloon can be used as a drug, it is not necessary to separately provide a container for the drug within the casing, thereby allowing the size of the observation device to be reduced.

The eighteenth invention is the in vivo observation device of the eleventh invention wherein, the fluid is an optically transparent liquid drug, and a duct that connects the outside and inside of the balloon, a switching valve that can open and close the duct and release drug inside the balloon to the outside, and a switching valve control section that controls operation of the switching valve, are provided in the casing.

In the in vivo observation device as claimed in this invention, a switching valve control section operates a switching valve so as to open a duct when the drug is released as a result of observation by the observation device or when a signal from outside the living body has been received. As a result, drug within the balloon is released outside the casing through the duct. In addition, the release of drug can be interrupted as a result of the switching valve control section closing the switching valve. In this manner, the drug can be reliably administered at the desired location of an affected area by opening and closing the switching valve. In addition, since the fluid that expands the balloon can be used as a drug, it is not necessary to separately provide a container for the drug within the casing, thereby allowing the size of the observation device to be reduced.

The nineteenth invention is the in vivo observation device of the eleventh invention wherein, a drug storage section that stores a drug, a duct that connects the drug storage section with the outside of the balloon, a releasing device interposed in the duct which releases drug stored in the drug storage section outside the balloon, and a control section that operates the releasing device, are provided in the casing.

In the in vivo observation device as claimed in this invention, the control section operates the releasing device, and drug housed in the drug storage section is released to outside the balloon through the duct when the drug is released as a result of observation by the observation device or when a signal from outside the living body has been received. In this manner, the drug can be reliably administered at the desired location of an affected area by the releasing device.

The twentieth invention is the in vivo observation device of the fourteenth invention wherein, a releasing device is provided within the balloon that releases the fluid outside the balloon when pressure within the balloon has reached a pressure equal to or greater than a predetermined value, the fluid is an optically transparent drug, and the control section operates the pump so that the pressure within the balloon reaches a pressure equal to or greater than a predetermined value.

In the in vivo observation device as claimed in this invention, the control section controls the operation of the pump so that the pressure within the balloon rises to a pressure equal to or greater than a predetermined value as a result of observation by the observation device or when a signal from outside the living body has been received.

The twenty-first invention is the in vivo observation device of the seventeenth invention wherein, another balloon is provided in the casing that has an electrode on its external surface and stores the drug inside, an electrical power supply section that supplies electrical power to the electrode and ducts that respectively communicate with the insides of the first balloon and the other balloon are provided within the casing, and the electrical power supply section supplies electrical power to the electrode to release the drug.

In the in vivo observation device as claimed in this invention, current is made to flow within the living body as a result of the electrical power supply section supplying electrical power to the electrode when drug is released as a result of observation by the observation device or for which a signal has been received from outside the living body. The living body contracts in response to this current stimulation and presses against the other balloon from the outside. In response to this pressing, drug housed within the other balloon moves inside the first balloon through a duct. As a result, the pressure inside the first balloon rises to a pressure equal to or greater than a predetermined value and the drug is released through micropores. In this manner, since an external force received from the living body can be effectively used to administer a drug, electrical power used during drug administration can be conserved and the drug can be administered efficiently.

The twenty-second invention is the in vivo observation device of the eleventh invention wherein, the refractive index of the balloon is equal to or lower than the refractive index of the fluid.

In the in vivo observation device as claimed in this invention, since the since the refractive index of the fluid is either equal to or higher than the refractive index of the balloon when the balloon is expanded by supplying a fluid inside the balloon, it is difficult for reflection to occur inside the balloon at the interface between the balloon and the fluid. Thus, satisfactory observation can be carried out.

The twenty-third invention is the in vivo observation device of the second invention wherein, the contact auxiliary device is the difference in specific gravity of the entire device such that the specific gravity of the entire device is set to be larger than the specific gravity of the fluid present in the body. As a result, the in vivo observation device having a specific gravity larger than the fluid can be made to closely adhere to body tissue by submerging in body fluid.

The twenty-fourth invention is the in vivo observation device of the twenty-third invention wherein, the center of gravity is decentered towards the observation wall surface. As a result, the observation wall surface that faces downward due to gravity can be reliably closely adhered to body tissue.

The twenty-fifth invention is the in vivo observation device of the third invention wherein, the action generating section is a pressing section that presses against the living body, and a grip for operating pressing is provided in the external contact auxiliary device. As a result, the ease of operating the pressing operation that causes the casing to press against body tissue by applying pressure from outside the living body is improved.

The twenty-sixth invention is the in vivo observation device of the twenty-fifth invention wherein, a transmission device that transmits data is provided within the casing, and a display section that displays data transmitted from the transmission device is provided in the pressing section. As a result, the device can be operated while confirming the degree of oppression by viewing images displayed on the display section.

The twenty-seventh invention is the in vivo observation device of the twenty-fifth invention wherein, a location detecting device that detects the location of the casing is provided in the external contact auxiliary device. As a result, the pressing operation can be carried out by accurately determining the location of the casing.

The twenty-eighth invention is the in vivo observation device of the twenty-fifth invention wherein, a permanent magnet for electromagnetic attraction and an antenna for receiving electrical power are provided within the casing, and a coil for magnetic attraction and an antenna for supplying electrical power are provided in the pressing section. As a result, the in vivo observation device can be made to closely adhere to body tissue by the electromagnetic attraction action produced between the permanent magnet and coil for magnetic attraction. In addition, energy can also be supplied to the casing within the living body by the power generation action of the antenna for supplying electrical power and the antenna for receiving electrical power. In this case, if the antenna for receiving electrical power is arranged between the S and N poles according to the orientation of the magnetic poles, namely in consideration of the arrangement of the magnetic poles (S and N poles) that mutually attract, power generation efficiency can be increased by directly opposing the antenna for supplying electrical power.

The twenty-ninth invention is the in vivo observation device of the fourth invention wherein, the action generating section is a magnetic field generating device, and the reacting section is a permanent magnet or a ferromagnetic body. As a result, the casing can be magnetically attracted and closely adhered to body tissue by an electromagnet simultaneous to applying oppression from outside the living body with an oppressor. In this case, the electromagnetic is preferably able to be switched on and off as necessary.

The thirtieth invention is the in vivo observation device of the twenty-ninth invention wherein, a transmission device that transmits data is provided within the casing, and a display section that displays data transmitted from the transmission device is provided in the pressing section. As a result, the device can be operated while confirming the degree of oppression by viewing images displayed on the display section.

The thirty-first invention is the in vivo observation device of the twenty-ninth invention wherein, a location detecting device that detects the location of the casing is provided in the external contact auxiliary device. As a result, the pressing operation can be carried out by accurately determining the location of the casing.

The thirty-second invention is the in vivo observation device of the twenty-ninth invention wherein, a permanent magnet for electromagnetic attraction and an antenna for receiving electrical power are provided within the casing, and a coil for magnetic attraction and an antenna for supplying electrical power are provided in the pressing section. As a result, effects similar to those of the twenty-eighth invention are obtained.

The thirty-third invention is the in vivo observation device of the first invention wherein, a drug administration device is provided that administers a drug at a desired site from the casing when the casing is closely contacted to the body tissue. As a result, a drug can be accurately administered to body tissue at a target site.

The following describes specific examples of this type of drug administration device.
(1) A drug is impregnated into a sponge-like drug storage/transport member. When pressure is applied to the drug storage/transport member that has closely adhered to body tissue at a desired site, the drug is pushed out and coated onto body tissue of the affected area.
(2) A plurality of minute needles are provided in a drug storage/transport member. When pressure is applied to the drug storage/transport member when the needles have punctured body tissue as a result of being closely adhered thereto, the drug is pushed out and injected directly into body tissue of the affected area.
(3) A gel-like substance in which a drug has been impregnated is stored in a drug storage/transport device. When this substance is released and adheres to body tissue of an affected area, it remains on the affected area for a long period of time, thereby allowing the drug to be released gradually.
(4) When a gel-like drug stored in a drug storage/transport device is led to the surface by electrophoresis, it can be applied to body tissue which it is closely adhered over a long period of time and over a wide range.
(5) When a drug stored in a drug storage/transport device is released after being atomized, it can be administered over a wide range of body tissue. In this case, since the drug can be administered over an even wider range by pressure release if the atomized drug is pushed out by compressing, this is particularly suitable for long organs like the intestines.
(6) It is preferable to provide a foreign object removal device that removes body fluid and so forth present on the surface of an affected area by releasing air and so forth prior to drug administration, and as a result, body tissue of the affected area can be exposed enabling the drug to be administered reliably. In this case, the drug is preferably administered automatically in the order of foreign object removal followed by administration.

In addition, the field of view of the previously described observation system may be secured by removing foreign objects around an affected area by using this foreign object removal device.

The thirty-fourth invention provides an examination method comprising: a step in which a in vivo observation device is introduced into a subject, a step in which the location of in vivo observation device within a living body is recognized, a step in which the in vivo observation device is closely contacted to a body wall based on the recognized location, and a step in which an image of the closely contacted section is acquired.

The thirty-fifth invention is the thirty-fourth invention
wherein, the step in which the location within the living body is recognized comprises recognizing with an image acquired by the in vivo observation device.

The thirty-sixth invention is the thirty-fourth invention
wherein, the step in which the location within the living body is recognized comprises recognizing according to a timer installed in the in vivo observation device.

The thirty-seventh invention is the thirty-fourth invention wherein, the step in which the location within the living body is recognized comprises recognizing based on location information of the in vivo observation device.

The thirty-eighth invention is the thirty-fourth invention
wherein, the step in which the in vivo observation device is closely contacted to a body wall comprises closely contacting by a fluid transfer device that aspirates a fluid such as body fluid or gas in the living body from the front in the direction of observation of the in vivo observation device and causes it to flow out to the rear.

The thirty-ninth invention is the thirty-fourth invention
wherein, the step in which the in vivo observation device is closely contacted to a body wall comprises closely contacting a balloon to a body wall by supplying a fluid to an optically transparent balloon provided in the observation section in the in vivo observation device to expand the balloon.

The fortieth invention is the thirty-fourth invention
wherein, the step in which the in vivo observation device is closely contacted to a body wall is a step in which pushing pressure is applied from outside the living body to a portion where the in vivo observation device is present.

The forty-first invention is the thirty-fourth invention
wherein, the step in which the in vivo observation device is closely contacted to a body wall comprises closely contacting by generating a magnetic field outside the living body and attracting a magnet or ferromagnetic body installed within the in vivo observation device.

The forty-second invention provides an examination and treatment method comprising: a step in which a in vivo observation device is introduced into a subject, a step in which the location within a living body is recognized, a step in which the in vivo observation device is closely contacted to a body wall based on the recognized location, a step in which an image of the closely contacted section is acquired, a step in which the acquired image is confirmed, and a step in which a drug is released if necessary.

The forty-third invention is the forty-second invention
wherein, the step in which the location within the living body is recognized comprises recognizing with an image acquired by the in vivo observation device.

The forty-fourth invention is the forty-second invention
wherein, the step in which the location within the living body is recognized comprises recognizing according to a timer installed in the in vivo observation device.

The forty-fifth invention is the forty-second invention
wherein, the step in which the location within the living body is recognized comprises recognizing based on location information of the in vivo observation device.

The forty-sixth invention is the forty-second invention
wherein, the step in which the in vivo observation device is closely contacted to a body wall comprises closely contacting by a fluid transfer device that aspirates a fluid such as body fluid or gas in the living body from the front in the direction of observation of the in vivo observation device and causes it to flow out to the rear.

The forty-seventh invention is the forty-second invention
wherein, the step in which the in vivo observation device is closely contacted to a body wall comprises closely contacting a balloon to a body wall by supplying a fluid to an optically transparent balloon provided in the observation section in the in vivo observation device to expand the balloon.

The forty-eighth invention is the forty-second invention
wherein, the step in which the in vivo observation device is closely contacted to a body wall is a step in which pushing pressure is applied from outside the living body to a portion where the in vivo observation device is present.

The forty-ninth invention is the forty-second invention
wherein, the step in which the in vivo observation device is closely contacted to a body wall comprises closely contacting by generating a magnetic field outside the living body and attracting a magnet or ferromagnetic body installed within the in vivo observation device.

According to the in vivo observation device of the present invention, since body tissue can be observed through a balloon by expanding the balloon causing it to be closely adhered to body tissue, the state of body tissue can be reliably observed without flushing the intestines. In the case of inflammatory bowel diseases in particular, the state of the digestive tract that contains an affected area can be reliably observed while preventing exacerbation of symptoms caused by flushing the intestines.

In addition, according to the in vivo observation device of the present invention, since body tissue can be observed in a state in which the observation wall surface and body tissue are closely adhered by a contact auxiliary device, the state of body tissue can be reliably observed without flushing the intestines and without obstruction of the field of view by body fluids, gases and other fluids. In cases of inflammatory bowel diseases in particular, the state of the digestive tract that contains an affected area can be reliably observed while preventing exacerbation of symptoms caused by flushing the intestines.

### BRIEF DESCRIPTION OF THE DRAWINGS

Fig. 1 is a cross-sectional view showing a first embodiment of an in vivo observation device as claimed in the present invention.
Fig. 2 is a cross-sectional view of an in vivo observation device showing the state in which a balloon of the in vivo observation device shown in Fig. 1 is expanded and closely contacted to body tissue.
Fig. 3 is a cross-sectional view showing a second embodiment of an in vivo observation device as claimed in the present invention in the state in which a balloon is expanded and closely contacted to body tissue.
Fig. 4 is a cross-sectional view showing a third embodiment of an in vivo observation device as claimed in the present invention in the state in which a balloon is expanded and closely contacted to body tissue.
Fig. 5 is a cross-sectional view showing a fourth embodiment of an in vivo observation device as claimed in the present invention in the state in which a balloon is expanded and closely contacted to body tissue.
Fig. 6 is a cross-sectional view showing the state in which another balloon of the in vivo observation device shown in Fig. 5 is expanded and closely contacted to body tissue.
Fig. 7 is a side view showing a fifth embodiment of an in vivo observation device as claimed in the present invention in the state in which a balloon is expanded.
Fig. 8 is a cross-sectional view showing a sixth embodiment of an in vivo observation device as claimed in the present invention in the state in which a balloon is expanded and closely contacted to body tissue.
Fig. 9 is a cross-sectional view showing a seventh embodiment of an in vivo observation device as claimed in the present invention in the state in which a balloon is expanded and closely contacted to body tissue, and a drug is administered.
Fig. 10 is a cross-sectional view showing an eighth embodiment of an in vivo observation device as claimed in the present invention in the state in which a balloon is expanded and closely contacted to body tissue, and a drug is administered.
Fig. 11 is a cross-sectional view showing a ninth embodiment of an in vivo observation device as claimed in the present invention in the state in which a balloon is expanded and closely contacted to body tissue, and a drug is administered.
Fig. 12 is a cross-sectional view showing a tenth embodiment of an in vivo observation device as claimed in the present invention in the state in which a balloon is expanded and closely contacted to body tissue, and a drug is administered.
Fig. 13 is a cross-sectional view showing an eleventh embodiment of an in vivo observation device as claimed in the present invention in the state in which a first balloon and another balloon are expanded and closely contacted to body tissue, and a drug is administered.
Fig. 14 is a cross-sectional view showing another example of an in vivo observation device as claimed in the present invention in the state in which a balloon is expanded and closely contacted to body tissue.
Fig. 15 is a cross-sectional view showing still another example of an in vivo observation device as claimed in the present invention in the state in which a balloon is expanded and closely contacted to body tissue.
Fig. 16 is a cross-sectional view showing a twelfth embodiment of an in vivo observation device as claimed in the present invention.
Fig. 17 is an explanatory drawing of the operation of the in vivo observation device shown in Fig. 16, with Fig. 17A showing the state in which a pump body has begun to operate in the forward direction, Fig. 17B showing the state of forward operation of the pump body, Fig. 17C showing the state of closely adhering to body tissue by suctioning, and Fig. 17D showing the state of the pump body has been operated in the reverse direction.
Fig. 18 is a cross-sectional view showing a variation of the twelfth embodiment shown in Fig. 16.
Fig. 19 is a cross-sectional view showing a thirteenth embodiment of an in vivo observation device as claimed in the present invention.
Fig. 20 is a cross-sectional view showing a variation of the thirteenth embodiment shown in Fig. 19.
Fig. 21 is a cross-sectional view showing a fourteenth embodiment of an in vivo observation device as claimed in the present invention.
Fig. 22 is a cross-sectional view showing a variation of the fourteenth embodiment shown in Fig. 21.
Fig. 23 is a cross-sectional view showing a fifteenth embodiment of an in vivo observation device as claimed in the present invention.
Fig. 24 is a cross-sectional view showing a sixteenth embodiment of an in vivo observation device as claimed in the present invention.
Fig. 25 is an enlarged cross-sectional view of the essential portion showing a variation of the sixteenth embodiment shown in Fig. 24.
Fig. 26 is a side view showing a seventeenth embodiment of an in vivo observation device as claimed in the present invention.
Fig. 27 is a drawing showing a variation of the seventeenth embodiment shown in Fig. 26, with Fig. 27A being a perspective view showing the use of an oppression device, and Fig. 27B being a cross-sectional view showing an example of the constitution of the oppression device.
Fig. 28 is a cross-sectional view showing a first variation of an oppression device.
Fig. 29 is a drawing showing a second variation of an oppression device, with Fig. 29A being a cross-sectional view and Fig. 29B being an enlarged cross-sectional view of the essential portion.
Fig. 30 is a cross-sectional view showing a third variation of an oppression device.
Fig. 31 is a cross-sectional view showing an example of the constitution of a drug administration device as an eighteenth embodiment of an in vivo observation device as claimed in the present invention.
Fig. 32 is a cross-sectional view showing a first variation of the drug administration device shown in the eighteenth embodiment of Fig. 31.
Fig. 33 is a cross-sectional view showing a second variation of the drug administration device shown in the eighteenth embodiment of Fig. 31.
Fig. 34 is a cross-sectional view showing a third variation of the drug administration device shown in the eighteenth embodiment of Fig. 31.
Fig. 35 is a cross-sectional view showing a fourth variation of the drug administration device shown in the eighteenth embodiment of Fig. 31.
Fig. 36 is a cross-sectional view showing a fifth variation of the drug administration device shown in the eighteenth embodiment of Fig. 31.
Fig. 37 is a cross-sectional view showing a sixth variation of the drug administration device shown in the eighteenth embodiment of Fig. 31.
Fig. 38 is a cross-sectional view showing a seventh variation of the drug administration device shown in the eighteenth embodiment of Fig. 31.
Fig. 39 is a cross-sectional view showing an eighth variation of the drug administration device shown in the eighteenth embodiment of Fig. 31.

### BEST MODE FOR CARRYING OUT THE INVENTION

The following provides an explanation of preferable embodiments of the present invention with reference to the drawings. However, the present invention is not limited to these embodiments, and for example, the constituent features of these embodiments may be suitably combined.

### <First Embodiment>

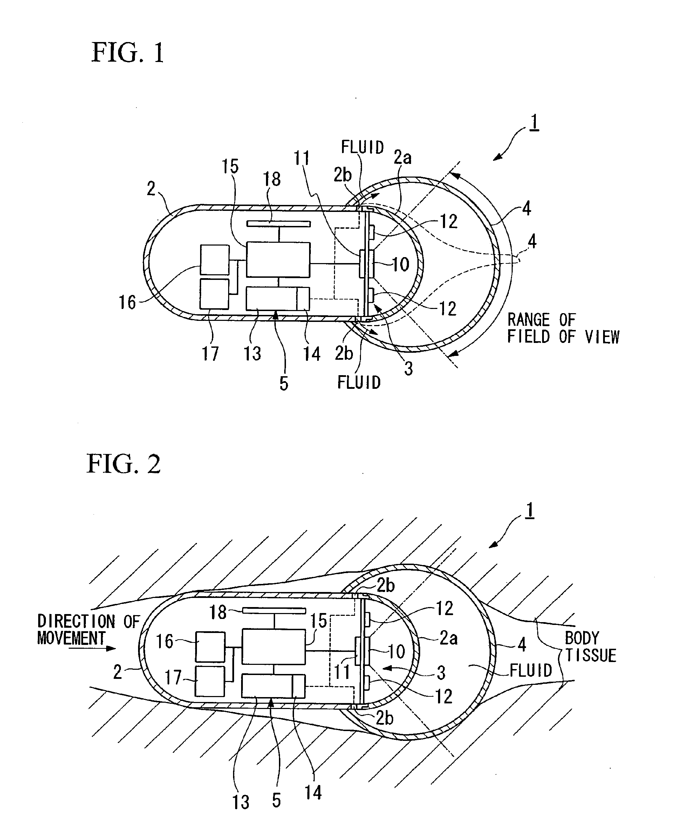

The following provides an explanation of a first embodiment of an in vivo observation device as claimed in the present invention with reference to Figs. 1 and 2. As shown in Fig. 1, in vivo observation device 1 of the present embodiment is provided with a capsular casing 2 that is taken orally into the body, an observation system (observation device) 3 provided within casing 2 that observes the inside of the body, an optically transparent balloon 4 provided in casing 2 so as to cover the periphery of said observation system 3 and which is able to be expanded so as to closely contact or adhere to body tissue when moved within the body, and an expansion device 5 that expands balloon 4 by supplying a fluid within said balloon 4.

Furthermore, refractive index nₐ of the transparent material of balloon 4 is either equal to or less than the refractive index n_{b} of the fluid supplied within balloon 4 (nₐ ≤ n_{b}).

The aforementioned casing 2 is formed so that the inside is sealed with plastic and so forth, and a transparent cover 2a is provided on one end. An object lens 10 that captures images of each part within the body is arranged on the inside of this transparent cover 2a, and a CMOS imager or other imaging element 11 is arranged at the image forming location of said object lens 10. In addition, an LED 12 that illuminates the periphery of the field of view of object lens 10 by emitting an illumination light is arranged around object lens 10. Namely, this object lens 10, imaging element 11, and LED 12 compose the aforementioned observation system 3.

The aforementioned balloon 4 is formed from an expandable, elastic material such as rubber, and the end is attached to the periphery of casing 2 so as to cover the entire transparent cover 2a. In other words, balloon 4 is arranged so as to cover the viewing angle of object lens 10, and observation system 3 is made to observe inside the body through balloon 4. In addition, a plurality of fluid supply ports 2b are formed around the axis of casing 2 for supplying the aforementioned liquid from a storage section 13 that houses fluid arranged within said casing 2 to the inside of balloon 4. These fluid supply ports 2b are connected to storage section 13 by ducts not shown. In addition, a switching valve 14 is provided in storage section 13, and fluid housed inside can be supplied into balloon 4 through a duct by opening and closing said switching valve 14. Namely, this storage section 13 and switching valve 14 compose the aforementioned expansion device 5.

Moreover, a control section 15 that controls the aforementioned observation system 3, a memory 16 that records captured images acquired with observation system 3, a judgment section 17 that judges whether or not in vivo observation device 1 has reached a predetermined site in the intestines, for example, based on captured images acquired with observation system 3, and a battery 18 that supplies electrical power to each of the aforementioned constituent parts, are provided within casing 2. Judgment section 17 has a function that transmits a signal to control section 15 when judgment section 17 judges that in vivo observation device 1 has reached the intestines. After having received that signal, control section 15 operates the aforementioned switching valve 14 and records a captured image acquired by observation system 3 in the aforementioned memory 16.

The following provides an explanation of the case of observing inside the body with an in vivo observation device 1 composed in this manner. Furthermore, in the present embodiment, detailed observations are set to be made by expanding balloon 4 when in vivo observation device 1 has reached the intestines.

An in vivo observation device 1 taken orally by a patient not shown moves through the body along the digestive tract.

Furthermore, a switch not shown is made to be turned on at this time, and electrical power is supplied from battery 18 to each constituent part. In addition, control section 15 operates observation system 3 so as to capture images inside the body.

Here, in the case in vivo observation device 1 has reached the intestines, judgment section 17 judges that it has reached the intestines in response to plicate tissue characteristic of the intestines having been confirmed in captured images, for example, based on images captured with observation system 3.

When judgment section 17 judges that in vivo observation device 1 has reached the intestines, it informs control section 15 of this. In response to this, said control section 15 operates switching valve 14 and performs control so as to record images captured with observation system 3 in memory 16.

On the other hand, switching valve 14, after having received a signal from control section 15, supplies fluid housed in storage section 13 to balloon 4 through fluid supply ports 2b with the duct open. As a result, balloon 4 is expanded and closely adhered to body tissue as shown in Fig. 2. At this time, even there were foreign objects such as body fluids or food residue between the outside of balloon 4 and the body, since balloon 4 expands while pushing aside these foreign objects due to the pressure during expansion, it closely adheres to body tissue in the state in which said foreign objects have been removed.

As a result, after expanding balloon 4, in addition to observation system 3 being able to observe body tissue in the optimum state without being affected by foreign objects in the body, clear captured images can be recorded in memory 16. In particular, since the density of balloon 4 decreases considerably when it is expanded, it becomes transparent enabling observation system 3 to obtain even clearer captured images.

In addition, since in vivo observation device 1 moves while maintaining the state in which foreign objects are removed from between balloon 4 and body tissue in the same manner as when moving through the body, observation system 3 is able to make observations along the inside of the intestines.

Moreover, since a comparison of the refractive indices of balloon 4 and the fluid reveals that the refractive index of the fluid is equal to or higher than that of balloon 4, it is difficult for reflection to occur into balloon 4 at the interface between balloon 4 and the fluid, thereby enabling satisfactory observation.

According to in vivo observation device 1 as described above, since body tissue can be observed through balloon 4 by expanding balloon 4 within the intestines so that it is closely adhered to body tissue, the status of body tissue can be reliably observed without having to flush out the intestines and without being affected by foreign objects. In the case the patient has a disease such as an inflammatory bowel disease in particular, the status of the intestines can be reliably observed while preventing exacerbation of symptoms caused by flushing the intestines. In addition, since observations can be made along the entire length of the intestines, observations can be reliably made even at locations which were difficult to observe by endoscopic examination, such as locations at a considerable distance from the anus.

Furthermore, although balloon 4 is expanded when it has reached the intestines in the present embodiment, the balloon may also be expanded when it has reached any desired location. In addition, although judgment section 17 judges whether or not in vivo observation device 1 has reached the intestines based on images captured with observation system 3, and balloon 4 is expanded according to said judgment during expansion of balloon 4, the present embodiment is not limited to this, but rather a constitution may be employed in which, for example, the location of in vivo observation device 1 is confirmed outside the body, a signal is sent when it has reached a desired location, and balloon 4 is expanded by an expansion device when said signal has been received. Moreover, control section 15 may be set at that time so as to operate observation system 3 when said signal has been received. This being the case, since observation system 3 can be operated only at a location desired to be observed, electrical power can be conserved. In addition, a timer may also be provided as a trigger for expanding the balloon.

### <Second Embodiment>

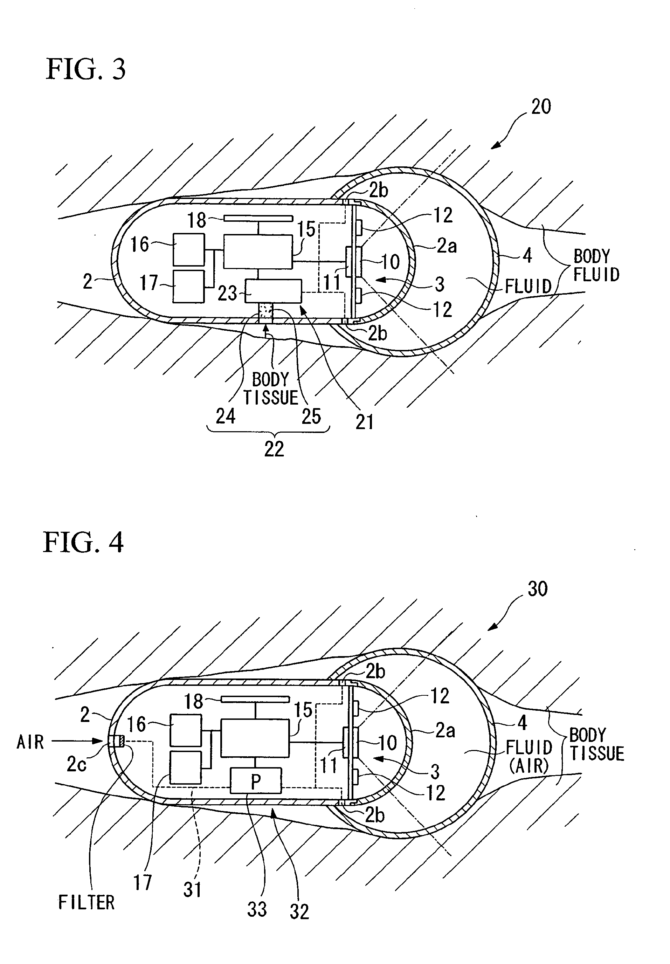

Next, an explanation is provided of a second embodiment of an in vivo observation device as claimed in the present invention with reference to Fig. 3.

Furthermore, the same reference symbols are used to indicate those constituent features in the second embodiment that are the same as those in the first embodiment, and their explanations are omitted.

The difference between the first embodiment and second embodiment is that, in contrast to fluid A housed in storage section 13 being supplied to balloon 4 by expansion device 5 when balloon 4 is expanded in the first embodiment, in vivo observation device 20 of the second embodiment expands balloon 4 using a body fluid in the body.

Namely, as shown in Fig. 3, in vivo observation device 20 of the present embodiment has an acquisition section 22 that acquires body fluid in the body, and an expansion tank (expansion section) 23 that expands balloon 4 based on the amount of moisture of the body fluid acquired with said acquisition section 22.

One end of said expansion tank 23 is connected to fluid supply ports 2b, and the other end is connected to duct 24 that communicates with the outside of casing 2. In addition, this duct 24 is filled with a polymer gel 25, and when this polymer gel 25 becomes saturated by gradually absorbing moisture from body fluid, the moisture that overflows is supplied to expansion tank 23. Namely, this duct 24 and polymer gel 25 compose the aforementioned acquisition section 22. Furthermore, after polymer gel 25 has become saturated with moisture, it solidifies after the passage of a predetermined amount of time thereby preventing any further acquisition of moisture.

The aforementioned expansion tank 23 contains a foaming agent such as granular carbonic acid that reacts with moisture, which together with generating a gas (fluid) such as carbon dioxide gas from the moisture supplied from polymer gel 25, supplies the generated gas to balloon 4 through fluid supply ports 2b.

Furthermore, in in vivo observation device 20 of the present embodiment, the amount, density and so forth of polymer gel 25 is adjusted so that moisture is supplied to expansion tank 23 when in vivo observation device 20 has reached the intestines.

In in vivo observation device 20 composed in this manner, since balloon 4 can be expanded by generating a gas (fluid) such as carbon dioxide gas using moisture of body fluid, it is not necessary to provide a fluid in casing 2 in advance. Thus, the size of casing 2 can be reduced. In addition, since the moisture content at which polymer gel 25 becomes saturated can be easily adjusted by adjusting the amount, density and so forth of polymer gel 25, balloon 4 can be reliably expanded at a desired location in the body.

Furthermore, although polymer gel 25 is adjusted so that balloon 4 is expanded when in vivo observation device 20 has reached the intestines in the present embodiment, polymer gel 25 may be adjusted so as to expand balloon 24 at any desired location similar to the aforementioned first embodiment.

### <Third Embodiment>

Next, an explanation is provided of a third embodiment of an in vivo observation device as claimed in the present invention with reference to Fig. 4.

Furthermore, the same reference symbols are used to indicate those constituent features in the third embodiment that are the same as those in the first embodiment, and their explanations are omitted.

The difference between the third embodiment and the first embodiment is that, in contrast to fluid A housed in storage section 13 being supplied to balloon 4 by expansion device 5 when balloon 4 is expanded in the first embodiment, in vivo observation device 30 of the third embodiment expands balloon 4 using air (fluid) in the body.

Namely, as shown in Fig. 4, in vivo observation device 30 of the present embodiment is provided with a duct 31 within casing 2 that connects the outside and inside of balloon 4, and expansion device 32 is provided with a pump 33 that expands or contracts balloon 4 by either supplying air to balloon 4 from the outside, namely from the body, or by discharging air from said balloon 4 into the body, by means of duct 31.

One end of the aforementioned duct 31 is connected to fluid supply ports 2b, while the other end is connected to fluid intake port 2c provided in casing 2.

In in vivo observation device 30 composed in this manner, control section 15 operates pump 33 after receiving a signal indicating that in vivo device 30 has arrived at the intestines from judgment section 17. Together with taking in air from the body through fluid intake port 2c, said pump 33 is able to supply air to balloon 4 through fluid supply ports 2b to expand said balloon 4. In this manner, since balloon 4 can be expanded by acquiring air from the body, the size of casing 2 can be further reduced.

In addition, balloon 4 can be contracted following completion of observation by discharging air from inside balloon 4 by operating pump 33, and observations can subsequently be made again by suitably expanding balloon 4. In this manner, since balloon 4 can be expanded only at a location desired to be observed, in addition to in vivo observation device 30 being able to move smoothly through the body, observations can be made efficiently. In addition, in order to secure a field of view for observation system 3, a filter and so forth is provided at an intermediate point in duct 31, and air or other fluid having increased transparency as a result of having removed foreign objects is supplied to balloon 4.

Furthermore, although balloon 4 is expanded by taking in air from the body in the present embodiment, balloon 4 may also be expanded by taking in body fluid.

### <Fourth Embodiment>

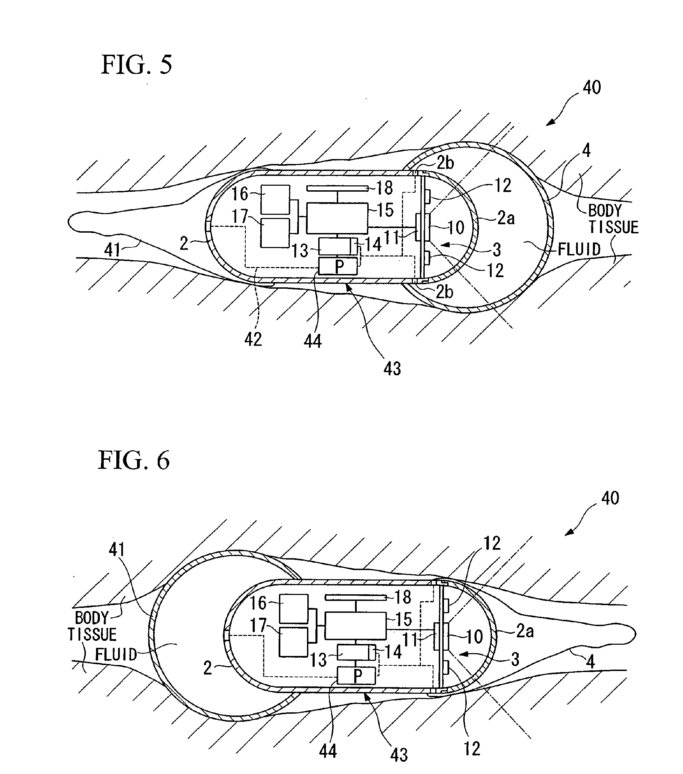

Next, an explanation is provided of a fourth embodiment of an in vivo observation device as claimed in the present invention with reference to Figs. 5 and 6. Furthermore, the same reference symbols are used to indicate those constituent features in the fourth embodiment that are the same as those in the first embodiment, and their explanations are omitted.

The difference between the fourth embodiment and the first embodiment is that, in contrast to fluid A housed in storage section 13 being supplied to balloon 4 by expansion device 5 when balloon 4 is expanded in the first embodiment, in in vivo observation device 40 of the fourth embodiment, another balloon 41 is arranged on the other end of casing 2 in addition to the aforementioned balloon 4, and a fluid is made to move back and forth between both balloons 4 and 41.

Namely, as shown in Fig. 5, in vivo observation device 40 of the present embodiment is provided with another balloon 41 in casing 2, and a duct 42 that respectively communicates with the insides of both balloons 4 and 41 within casing 2. In addition, an expansion device 43 has a pump 44 that mutually supplies a fluid to the insides of both balloons 4 and 41 by means of duct 42.

The aforementioned other balloon 41 is formed from an expandable, elastic material such as rubber in the same manner as balloon 4, and is attached to the periphery of casing 2 so as to be located on the opposite side of balloon 4.

In in vivo observation device 40 composed in this manner, control section 15 receives a signal indicating that in vivo observation device 40 has arrived at the intestines from judgment section 17, and supplies fluid A from storage section 13 to balloon 4 by operating switching valve 14 to expand balloon 4 and cause it to closely adhere to body tissue. Following completion of observation by observation system 3, balloon 4 is temporarily contracted until the next desired location is reached. Namely, control section 15 operates pump 44 to supply fluid within balloon 4 to the other balloon 41 through duct 42 by operating pump 44. As a result, as shown in Fig. 6, the other balloon 41 expands as balloon 4 contracts. In addition, in the case of making observations through balloon 4, pump 44 is operated again and balloon 4 is expanded by supplying fluid from the other balloon 41 to the inside of balloon 4.

As has been described above, since balloon 4 can be expanded and contracted by allowing fluid to move back and forth between both balloons 4 and 41, it is not necessary to replace the fluid. Thus, entry of foreign objects into the fluid can be prevented, and the field of view of observation system 3 can be maintained in the optimum state (clear field of view). In addition, in the case of desiring an enlarged view of a limited, narrow observation range, in vivo observation device 40 can be made to approach the observation site by operating pump 44 until balloon 4 closely adheres to transparent cover 2a.

Furthermore, although balloon 4 is expanded by supplying fluid housed in storage section 13 to balloon 4 when balloon 4 is initially expanded in the present embodiment, the present embodiment is not limited to this, but rather a constitution may also be employed in which, for example, in vivo observation device 40 may be taken orally into the body in the state in which a volume of fluid that fills balloon 4 is supplied in advance to balloon 4 and the other balloon 41.

This being the case, since it is not necessary to provide a storage section 13 and so forth in casing 2, the size of in vivo observation device 40 can be further reduced.

### <Fifth Embodiment>

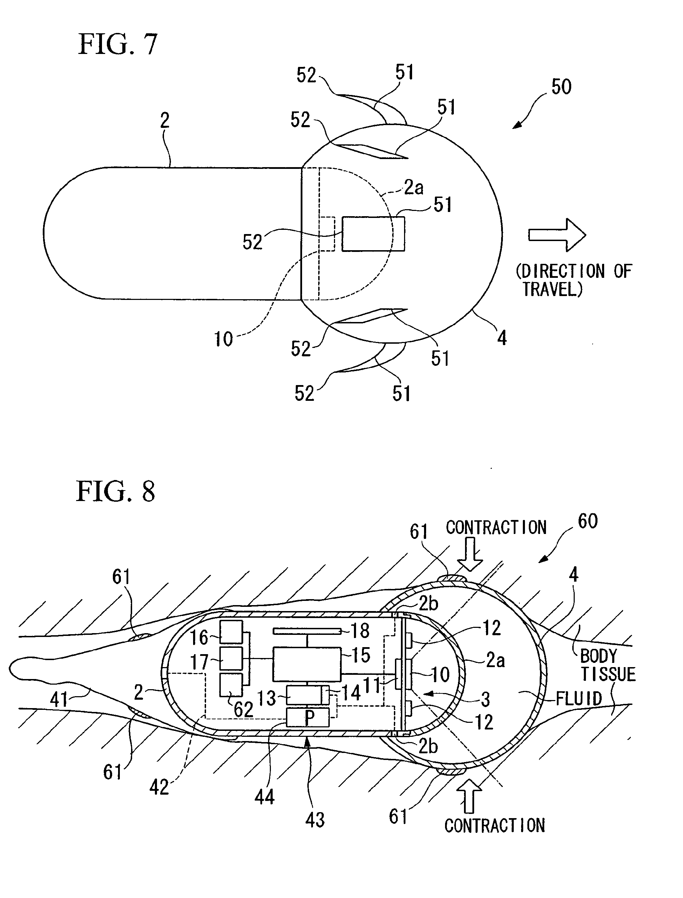

Next, an explanation is provided of a fifth embodiment of an in vivo observation device as claimed in the present invention with reference to Fig. 7.

Furthermore, the same reference symbols are used to indicate those constituent features in the fifth embodiment that are the same as those in the first embodiment, and their explanations are omitted.

The difference between the fifth embodiment and the first embodiment is that, in contrast to fluid A housed in storage section 13 being supplied to balloon 4 by expansion device 5 when balloon 4 is expanded in the first embodiment, in in vivo observation device 50 of the fifth embodiment, the direction of movement in the body is restricted by a reversing prevention device 51 provided in balloon 4.

Namely, as shown in Fig. 7, balloon 4 is provided with the aforementioned reversing prevention device 51 that has projections 52 on its outer surface which protrude towards the rear with respect to the direction of travel.

This reversing prevention device 51 is formed from an elastic material in the same manner as balloon 4, but has higher rigidity than said balloon 4. In addition, a plurality of reversing prevention devices 51 are provided around the axis of balloon 4 at locations outside the range of the field of view of observation system 3.

In in vivo observation device 50 composed in this manner, reversing prevention device 51 also closely adheres to body tissue when balloon 4 is expanded and made to closely adheres to body tissue. Although force is generated that pushes out in vivo observation device 50 towards the rear in the case contraction has occurred immediately in front of in vivo observation device 50 in particular, in this case as well, projection 52 prevents in vivo device 50 from reversing by engaging with body tissue to as to catch on said body tissue. Thus, in vivo observation device 50 is able to reliably move in the predetermined direction of travel and make suitable observations.

### <Sixth Embodiment>

Next, an explanation is provided of a sixth embodiment of an in vivo observation device as claimed in the present invention with reference to Fig. 8.

Furthermore, the same reference symbols are used to indicate those constituent features in the sixth embodiment that are the same as those in the fourth embodiment, and their explanations are omitted.

The difference between the sixth embodiment and the fourth embodiment is that, in contrast to the in vivo observation device simply moving by peristaltic movement, for example, during movement through the body, in vivo observation device 60 of the sixth embodiment is able to control movement through the body.

Namely, as shown in Fig. 8, in in vivo observation device 60 of the present embodiment, together with balloon 4 and another balloon 41 having electrodes 61 arranged on their outer surfaces, an electrical power supply section 62 is provided within casing 2 that supplies electrical power to electrodes 61. A plurality of the aforementioned electrodes 61 are provided around the axis of balloon 4 at locations outside the range of the field of view of observation system 3. In addition, control section 15 controls electrical power supply section 62 so that electrical power is supplied to electrodes 61 arranged on balloon 4 or 41 on the expanded side when balloon 4 or the other balloon 41 is expanded by operating pump 44.

In in vivo observation device 60 composed in this manner, electrodes 61 similarly closely adheres to body tissue when balloon 4 is expanded and made to closely adheres to body tissue. At this time, control section 15 supplies electrical power to electrodes 61 by operating electrical power supply section 62, causing electrical current to flow to the body tissue. Whereupon, the body tissue contracts as a result of being stimulated by the electrical current. This contraction primarily occurs in body tissue in the vicinity of the electrodes.

On the other hand, balloon 4 is moved towards the rear with respect to the direction of travel by an external force received from the outside due to the contraction of body tissue. In other words, balloon 4 moves in the opposite direction from the direction of movement through the body (e.g., direction of peristaltic movement). Thus, balloon 4 can be stopped at the same position to enable observations of longer duration and greater detail. In addition, since body tissue is stimulated by electrical current as a result of supplying electrical power to electrodes 61 when the other balloon 41 is expanded, propulsion in the direction of movement through the body can be promoted, thereby making it possible to increase the rate of movement through areas not requiring to be observed and allowing the in vivo observation device to reach the affected area quickly.

As has been described above, movement through the body can be controlled easily and reliably by promoting propulsion in the direction of travel or moving towards the rear with respect to the direction of travel.

### <Seventh Embodiment>

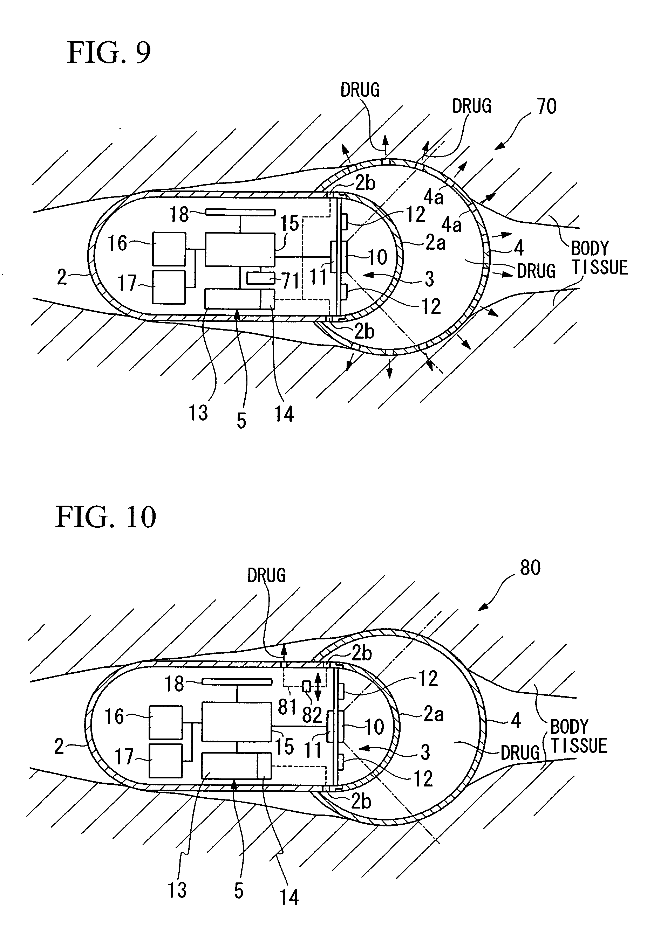

Next, an explanation is provided of a seventh embodiment of an in vivo observation device as claimed in the present invention with reference to Fig. 9.

Furthermore, the same reference symbols are used to indicate those constituent features in the seventh embodiment that are the same as those in the first embodiment, and their explanations are omitted.

The difference between the seventh embodiment and the first embodiment is that, in contrast to body tissue including an affected area being observed through balloon 4 in the first embodiment, in vivo observation device 70 of the seventh embodiment is able to administer a drug to an affected area in addition to observing that affected area.

Namely, as shown in Fig. 9, in in vivo observation device 70 of the present embodiment, the fluid is an optically transparent liquid drug, micropores 4a are provided in balloon 4 that discharge drug outside balloon 4 when said balloon 4 is expanded at a pressure equal to or greater than a predetermined value, and expansion device 5 has a pressure raising device 71 that raises the pressure inside balloon 4 to a pressure equal to or greater than a predetermined value during release of the drug.

A plurality of the aforementioned micropores 4a are provided over the entire outer surface of balloon 4. In addition, said micropores 4a are in a blocked state due to the elasticity of said balloon 4 until balloon 4 reaches the aforementioned pressure equal to or greater than a predetermined value, thereby preventing the drug from being released to the outside. The aforementioned pressure raising device 71 detects the pressure inside balloon 4 with a pressure sensor not shown, and supplies a fluid in the form of the drug to balloon 4 from storage section 13 by operating switching valve 14 when drug is released. Furthermore, in the present embodiment, judgment section 17 judges whether or not an affected area is the affected area that requires administration of the drug based on images captured by observation system 3.

According to in vivo observation device 70 composed in this manner, when observations are made by observation system 3 after expanding balloon 4 and causing it to closely adhere to body tissue, judgment section 17 judges an affected area to be the affected area that requires drug administration based on images captured by observation system 3 by judging, for example, that drug administration is required in the case a red color is detected in captured images and that red color exceeds a threshold value, and then informs pressure raising device 71 of that need for drug administration. After receiving this notification, said pressure raising device 71 expands balloon 4 by supplying drug to said balloon 4. At this time, pressure raising device 71 monitors the pressure inside balloon 4 with the pressure sensor, and interrupts expansion of balloon 4 by closing switching valve 14 when the pressure inside balloon 4 has reached a pressure equal to or greater than a predetermined value.

Since micropores 4a have been opened in balloon 4 that has been expanded to a pressure equal to or greater than a predetermined value by pressure raising device 71, the drug within balloon 4 is discharged to the outside through micropores 4a. At this time, since balloon 4 is closely adhered to the affected area, the discharged drug acts directly on the affected area without being affected by body fluid or other foreign objects. In this manner, together with allowing drug to be discharged at a desired location, pressure raising device 71 enables the drug to be reliably administered to an affected area without being affected by foreign objects. In addition, since the fluid that expands balloon 4 can be used for the drug, it is not necessary to separately provide a storage section for the drug within casing 2, thereby allowing the size of casing 2 to be reduced.

Furthermore, although a judgment as to whether drug is to be administered is made by judgment section 17 in the present embodiment, the present embodiment is not limited to this, but rather a constitution may be employed in which images captured by observation system 3 are monitored outside the body, a signal is sent when drug administration has been judged to be necessary based on said images, and pressure raising device 71 raises the pressure within balloon 4 when said signal is received.

### <Eighth Embodiment>

Next, an explanation is provided of an eighth embodiment of an in vivo observation device as claimed in the present invention with reference to Fig. 10. Furthermore, the same reference symbols are used to indicate those constituent features in the eighth embodiment that are the same as those in the seventh embodiment, and their explanations are omitted.

The difference between the eighth embodiment and the seventh embodiment is that, in contrast to drug being discharged through micropores 4a provided in balloon 4 in the seventh embodiment, in in vivo observation device 80 of the eighth embodiment, drug is discharged from balloon 4 to the outside through a duct 81.

Namely, as shown in Fig. 10, in vivo observation device 80 of the present embodiment is provided with the aforementioned duct 81 that connects the outside of balloon 4 with the inside, and a switching valve 82 that is able to open and close said duct 81 and release drug within balloon 4 to the outside, within casing 2.

Furthermore, in the present embodiment, control section 15 operates the aforementioned switching valve 82 when judgment section 17 has judged that an affected area is the affected area that requires administration of drug based on images captured with observation system 3. Namely, said control section 15 has the function of a switching valve control section that controls the operation of a switching valve.

According to in vivo observation device 80 composed in this manner, when judgment section 17 judges that an affected area is the affected area requiring administration of drug based on images captured by said observation system 3, control section 15 opens duct 81 by operating switching valve 82. As a result, drug inside balloon 4 is released outside balloon 4 through duct 81. In addition, administration of drug can be interrupted by closing switching valve 82. In this manner, the drug can be reliably administered at a desired location by opening and closing switching valve 82. In addition, since the fluid that expands balloon 4 is used for the drug, it is not necessary to separately provide a storage section for the drug within casing 2, thereby allowing the size of casing 2 to be reduced. Since the drug is released through duct 82 instead of administering the drug through balloon 4, there is no effect on observations by observation system 3 during said release. Thus, there is no decrease in observation performance.

Furthermore, in the present embodiment as well, although whether or not drug is administered is judged by judgment section 17 in the same manner as the aforementioned seventh embodiment, the present embodiment is not limited to this, but rather a constitution may be employed in which images captured by observation system 3 are monitored outside the body, a signal is sent when drug administration has been judged to be necessary based on said images, and switching valve 82 is operated when said signal is received.

### <Ninth Embodiment>

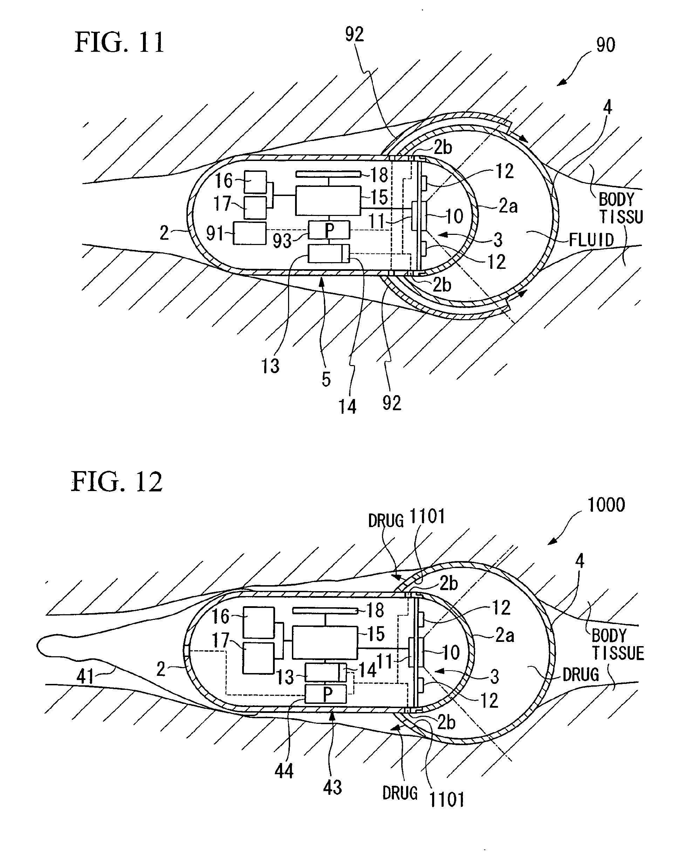

Next, an explanation is provided of a ninth embodiment of an in vivo observation device as claimed in the present invention with reference to Fig. 11. Furthermore, the same reference symbols are used to indicate those constituent features in the ninth embodiment that are the same as those in the seventh embodiment, and their explanations are omitted.

The difference between the ninth embodiment and the seventh embodiment is that, in contrast to drug being discharged through micropores 4a provided in balloon 4 in the seventh embodiment, in in vivo observation device 90 of the ninth embodiment, drug housed in a drug tank 91 (drug storage section) is released to the outside of balloon 4 through ducts 92.

Namely, in vivo observation 90 of the present embodiment is provided with the aforementioned drug tank 91 that houses a drug in casing 2, the aforementioned ducts 92 that connect said drug tank 91 with the outside of balloon 4, and a pump (releasing device) 93 that releases drug housed in drug tank 91 outside balloon 4 through said ducts 92. Furthermore, a fluid is supplied to balloon 4 in the same manner as in the first embodiment.

The aforementioned ducts 92 are composed of the same material as balloon 4, and are arranged so as to follow the outer periphery of balloon 4, opening outside casing 2 by passing through said casing 2 from drug tank 91. Namely, ducts 92 are integrally provided with balloon 4 on the outer surface of said balloon 4. In addition, the outlets of ducts 92, namely drug release outlets, are arranged at locations so as to be within the range of the observation field of view of observation system 3. Furthermore, in the present embodiment, control section 15 controls the operation of the aforementioned pump 93.

In in vivo observation device 90 composed in this manner, when judgment section 17 judges that an affected area is the affected area requiring drug administration based on images captured by said observation system 3, control section 15 operates pump 93 and causes drug housed in drug tank 91 to be released outside balloon 4 from the drug release outlets through ducts 92. At this time, since the drug can be administered while confirming the state of drug release with observation system 3, the drug can be administered accurately and efficiently.

Furthermore, in the present embodiment as well, although whether or not it is necessary to administer the drug is judged by judgment section 17, the present embodiment is not limited to this, but rather a constitution may be employed in which pump 93 is operated by receiving a signal and so forth from outside the body.

### <Tenth Embodiment>

Next, an explanation is provided of a tenth embodiment of an in vivo observation device as claimed in the present invention with reference to Fig. 12. Furthermore, the same reference symbols are used to indicate those constituent features in the tenth embodiment that are the same as those in the fourth embodiment, and their explanations are omitted.

The difference between the tenth embodiment and the fourth embodiment is that, in contrast to an affected area being observed through balloon 4 in the fourth embodiment, in vivo observation device 200 of the tenth embodiment also administers a drug to an affected area in addition to observing said affected area.

Namely, in in vivo observation device 1000 of the present embodiment, the fluid is an optically transparent liquid drug, a leak valve (releasing device) 1101 is provided within balloon 4 that releases the drug outside balloon 4 when the pressure of said balloon 4 has reached a pressure equal to or greater than a predetermined value, and control section 15 operates a pump 44 so that the pressure within balloon 4 reaches a pressure equal to or greater than a predetermined value during release of the drug. Furthermore, control section 15 detects the pressure within balloon 4 with a pressure sensor not shown.

In in vivo observation device 1000 composed in this manner, when judgment section 17 judges that an affected area is the affected area requiring drug administration based on images captured by said observation system 3, control section 15 expands balloon 4 by operating pump 44 and supplying drug within the other balloon 41 in said balloon 4. At this time, control section 15 monitors the pressure within balloon 4 with a pressure monitor, and stops pump 44 when the pressure within balloon 4 has reached a pressure equal to or greater than a predetermined value.

Once having been expanded to a pressure equal to or greater than a predetermined value, leak valve 1101 opens and the drug inside balloon 4 is discharged to the outside by said leak valve 1101. At this time, since balloon 4 is closely contacted or adhered to the affected area, the discharged drug acts directly on the affected area without being affected by body fluids or other foreign objects. In this manner, a drug can be reliably administered to an affected area at a desired location.

Furthermore, in the present embodiment as well, although judgment section 17 judges whether or not a drug is to be administered in the same manner as the aforementioned seventh embodiment, the present embodiment is not limited to this, but rather a constitution may be employed in which pump 33 is operated by receiving a signal and so forth from outside the body.

### <Eleventh Embodiment>

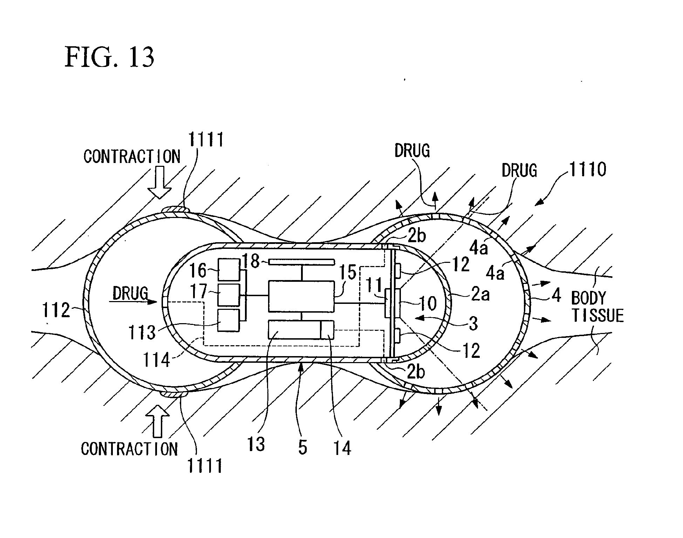

Next, an explanation is provided of an eleventh embodiment of an in vivo observation device as claimed in the present invention with reference to Fig. 13. Furthermore, the same reference symbols are used to indicate those constituent features in the eleventh embodiment that are the same as those in the seventh embodiment, and their explanations are omitted.

The difference between the eleventh embodiment and the seventh embodiment is that, in contrast to drug being discharged through micropores 4a by a pressure raising device 71 provided in balloon 4, in in vivo observation device 1110 of the eleventh embodiment, a drug is discharged from micropores 4a using an external force from the body.

Namely, in vivo observation device 1110 of the present embodiment is provided with another balloon 1112 inside casing 2 that has electrodes 1111 on its outer surface and which houses a drug inside, an electrical power supply section 1113 within casing 2 that supplies electrical power to electrodes 1111, and a duct 1114 that communicates with the inside of balloon 4 and the other balloon 1112, respectively. In addition, electrical power supply section 1113 supplies electrical power to electrodes 1111 when drug is released.

In the present embodiment, when judgment section 17 judges that an affected area is the affected area that requires administration of drug based on images captured by observation system 3, control section 15 controls electrical power supply section 113 so that electrical power is supplied to electrodes 1111.

In in vivo observation device 1110 composed in this manner, when judgment section 17 judges that it is necessary to administer the drug, control section 15 is informed of that need to administer drug. In response to this, said control section 15 operates electrical power supply section 1113 to supply electrical power to electrodes 1111 and cause electrical current to flow to the body tissue. As a result, the body tissue contracts in response to this stimulation and pushes on the other balloon 1112 from the outside. As a result of this pushing, drug housed inside the other balloon 1112 moves to the inside of balloon 4 through duct 1114. Furthermore, at this time, electrical power supply section 1113 supplies electrical current to electrodes 1111 so that the pressure inside balloon 4 reaches a pressure equal to or greater than a predetermined value according to a pressure sensor not shown that detects the pressure inside balloon 4.

On the other hand, since drug is supplied from the other balloon 1112, balloon 4 reaches a pressure equal to or greater than a predetermined value and micropores 4a open. As a result, since the drug inside balloon 4 is discharged to the outside through micropores 4a, drug can be administered to the affected area. In this manner, since a drug can be administered by effectively using external force received from the body, in addition to being able to conserve on electrical power during drug administration, the drug can be administered efficiently.

Furthermore, the technical field of the present invention is not limited to the aforementioned embodiments, but rather various modifications can be added within a range that does not deviate from the scope or spirit of the present invention.

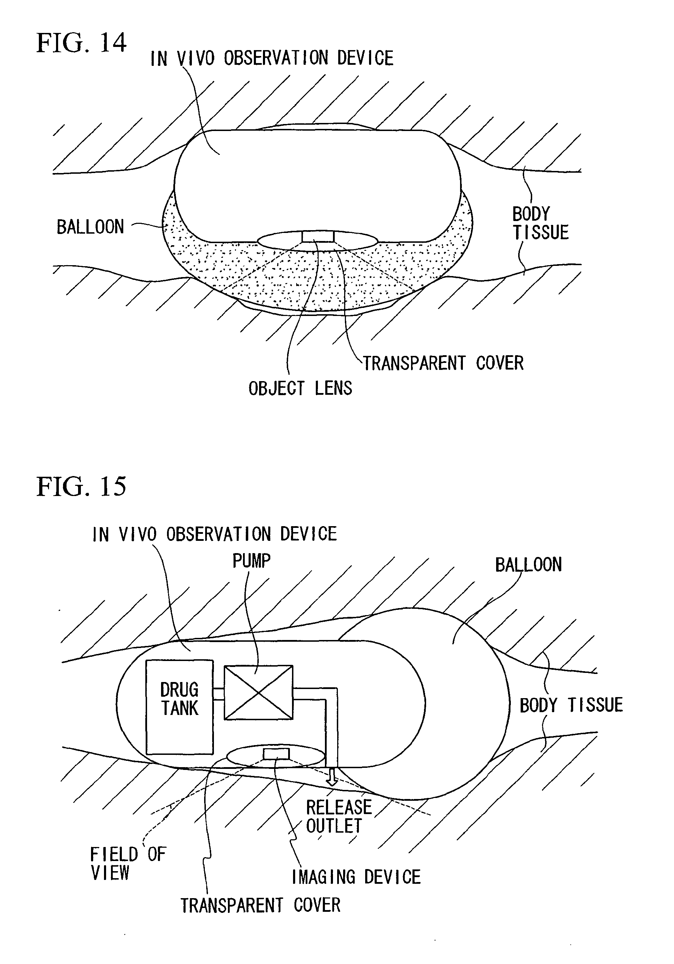

For example, although an observation system is provided on the end of a casing and observations are made towards the front in the direction of travel through the body in the aforementioned embodiments, the present invention is not limited to this, but rather a constitution may be employed in which the observation system may be arranged at any location of the casing provided observations can be made through the balloon. For example, a constitution may be employed in which the observation system is provided on the other end of the casing, and observations are made to the rear with respect to the direction of travel, or as shown in Fig. 14, the observation system may be provided on the side of the casing. In this case, since the observation system is arranged in opposition to the digestive tract, more detailed observations can be made of the mucosal tissue. Furthermore, at this time, a transparent cover and so forth should be provided on the casing that extends over the range of the field of view of the object lens of the observation system.

In addition, although the case of administering a drug in addition to observing the inside of the body by the observation system was indicated in the aforementioned seventh through eleventh embodiments, this is not limited to administration of a drug, but rather other functions may also be added based on observing through the balloon. For example, a function may be provided such as an acquisition device that acquires pH values, microbes or blood and so forth in the body.

In addition, in the case of adding another function in addition to observation in this manner, in the case of administering a drug, for example, the drug can be accurately administered at a desired location within the body by using the balloon. For example, in the in vivo observation device shown in Fig. 15, the observation system (imaging device) is arranged so as to face toward the side of the casing. Furthermore, a transparent cover is provided on the casing that extends over the range of the field of view of the imaging device. In addition, a drug tank that houses a drug, a duct that connects said drug tank with the outside of the casing, and a pump that discharges the drug inside the drug tank to outside the casing through said duct are provided within the casing.

Furthermore, a duct outlet, or in other words, a drug release outlet, is provided adjacent to the observation system.

In this in vivo observation device, when the in vivo observation device has reached the drug administration site, the balloon is expanded and closely adhered to body tissue and the in vivo observation device is immobilized in the body. Following this immobilization, a pump is operated and the drug is released outside the casing. Whereupon, drug can be precisely released at a desired location. Since the drug release outlets are adjacent to the observation system in particular, the status of drug administration can be confirmed with the observation system, thereby improving the accuracy of drug administration.

Moreover, in addition to the aforementioned constitutions, another balloon may be provided so that balloons are arranged on both sides of the casing. In this case, since a drug is administered after expanding both balloons, the drug can be retained between both balloons, and a large amount of drug can be released at a target site. In addition, even in cases in which the lumen is not oriented horizontally, since the shape is such that the drug is trapped between both balloons, flow of drug in the direction of gravity can be prevented thereby enabling the drug to be administered efficiently.

### <Twelfth Embodiment>

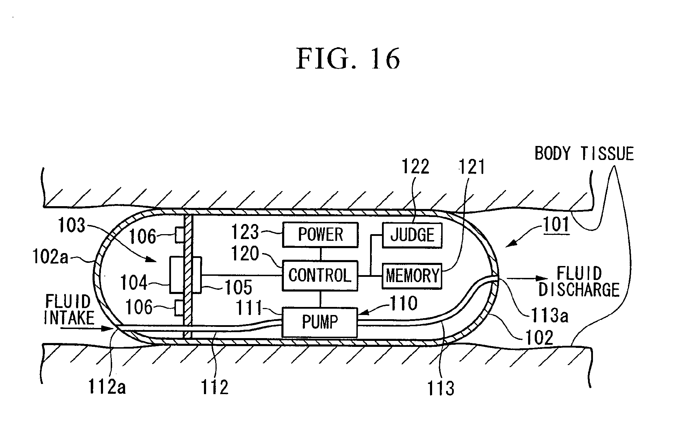

As shown in Fig. 16, in vivo observation device 101 of the present embodiment is equipped with a capsular casing 102 that is taken orally into the body, and an observation system (observation unit) 103 that observes the inside of the body through an optically transparent observation wall surface 102a provided in this casing 102, and a suction pump device (contact auxiliary unit) 110 of a fluid transfer unit is provided within casing 102 that causes observation wall surface 102a to closely adhere to body tissue during observation.

The aforementioned casing 102 is formed so that the inside is sealed with plastic and so forth, and observation wall surface 102a composed of a transparent material is provided in the shape of a cover on at least one end. An object lens 104 is arranged inside this observation wall surface 102a that captures images of each part within the body, and a CMOS imager or other imaging element 105 is arranged at the image forming location of said object lens 104. In addition, an LED 106 that illuminates the periphery of the field of view of object lens 104 by emitting an illumination light is arranged around object lens 104. Namely, this object lens 104, imaging element 105, and LED 106 compose the aforementioned observation system 103.

The aforementioned suction pump device 110 is composed by being equipped with a pump body 111 provided with a drive source and valves not shown, and ducts 112 and 113 connected upstream and downstream from said pump body 111.

Pump body 111 has a function that causes a liquid, gas or other fluid (to be referred to as "body fluid") to be aspirated through duct 112 opening on the front end of casing 102 provided with observation wall surface 102a to flow towards the rear through duct 113 opening on the rear end of casing 102. As a result, since suction pump device 110 discharges body fluid present in front of casing 102 from opening 113a in the rear by aspirating the fluid through opening 112a, observations can be made towards the front of casing 102 in a state in which body tissue is aspirated and closely adhered to observation wall surface 102a. The phenomenon in which body tissue is aspirated and closely adhered to observation wall surface 102a in this manner is effective in comparatively narrow, tubular organs like the intestines, and particularly in tubular organs of which the end is blocked.

Furthermore, the aforementioned suction pump device 110 is also able to cause body fluid to flow from opening 113a in the rear end of casing 102 to opening 112a in the front end by operating pump body 111 in reverse.

Moreover, a control section 120 that controls the aforementioned observation system 103, a memory 121 that records images acquired with observation system 103, a judgment section 122 that judges whether or not in vivo observation device 101 has reached a predetermined site such as in the intestines based on the images acquired with observation system 103, and a battery 123 provided as a power source that supplies electrical power to each of the aforementioned constituent parts, are provided within casing 102.

Judgment section 122 has a function by which, when in vivo observation device 101 is judged to have reached the intestines, it sends a signal to that effect to control section 120. After having received that signal, control section 120 operates the aforementioned suction pump device 110 and records images acquired by observation system 103 in the aforementioned memory 121.

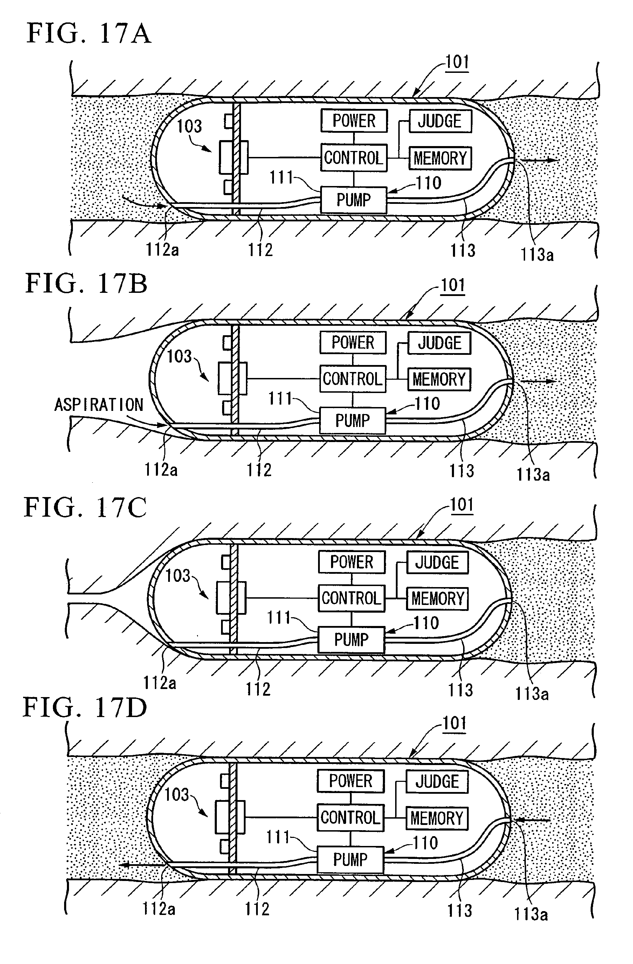

The following provides an explanation of the case of making observations in the body with in vivo observation device 110 composed in this manner with reference to Fig. 17. Furthermore, in the present embodiment, in vivo observation device 110 is set so that, when it reaches the location in the intestines where observations are to be made, suction pump device 110 is operated and body tissue is closely adhered to observation wall surface 102a to make detailed observations.

In vivo observation device 101 taken orally by a patient not shown moves through the body along the digestive tract.

Furthermore, a switch not shown is made to be turned on at this time, and electrical power is supplied from battery 123 to each constituent part. In addition, control section 120 operates observation system 3 so as to capture images inside the body.

Here, in the case in vivo observation device 101 has reached the intestines, judgment section 122 judges that it has reached the intestines in response to plicate tissue characteristic of the intestines having been confirmed in captured images, for example, based on images captured with observation system 103.

When judgment section 122 judges that in vivo observation device 101 has reached the intestines, it emits a signal to that effect to inform control section 120. In response to this signal, said control section 120 operates suction pump device 110 and performs control so as to record images captured with observation system 103 in memory 121.

Initially, suction pump device 110 is operated by a control signal from control section 120. As shown in Fig. 17A, suction pump device 110 in this case is operated in the forward direction so as to discharge body fluid aspirated from the front of casing 102 to the rear. Thus, body fluid present in front of observation wall surface 102a (front in the direction of observation) is subjected to suction force generated by the operation of pump body 111 and flows into duct 112 through opening 112a. After this body fluid has been led into pump body 111 through duct 112, it is then discharged from pump body 111, passes through duct 113 and is discharged to the rear of casing 102 from opening 113a. At this time, food residue and other foreign objects present in the intestines are also discharged to the rear of the casing together with the flow of body fluid.

As shown in Fig. 17B, as a result of the aforementioned operation of suction pump device 110 continuing, together with the body fluid present in the front in the direction of observation of in vivo observation device 101 decreasing, the inner wall of the intestine is suctioned in the direction of opening 112a by negative pressure. As a result, as shown in Fig. 17C, since body tissue of the intestinal wall closely adheres to observation wall surface 102a in which opening 112a is provided, observation system 103 is able to directly observe the body tissue based on a satisfactory field of view that is not obstructed by body fluid, food residue or other foreign objects. As a result of being able to observe the body tissue in this closely adhered state, clear captured images can be recorded in memory 121.

Following completion of observation, body fluid that has been discharged to the rear of casing 102 is returned to the front by reversing the operation of pump body 111. The instructions for reversing the operation of pump body 111 in this manner can be provided by, for example, transmitting a radio signal from outside the body to control section 120 and so forth in casing 102.

As an example of another means of providing the aforementioned instructions for reversing operation of pump body 111 in addition to a radio signal, a pressure sensor 116 may be contained at a suitable location in pump body 111, and the pressure detected by that pressure sensor 116 may be used as a means of providing the instructions described above. In this case, pump body 111 may be made to switch automatically from forward operation to reverse operation when a high pressure equal to or greater than a predetermined value is detected by pressure sensor 116, or valves not shown may open when the forward operation of pump body 111 stops, and body fluid may be allowed to flow in a communicating state between opening 112a and opening 113a. Furthermore, the location where pressure sensor 116 is installed is not limited to within pump body 111, but rather it may also be installed at an intermediate location of duct 112 or 113.

In addition, the forward operation of pump body 111 may be stopped or forward operation may be switched to reverse operation by judging that body tissue has been suctioned and has moved close to being closely adhered to observation wall surface 102a when image brightness has increased to a level equal to or greater than a predetermined value (threshold value) as a result of detecting the brightness of images captured with observation system 103.

According to in vivo observation device 101 described above, since body tissue can be observed directly from observation wall surface 102 by closely adhering the body tissue to be observed to observation wall surface 102a by operating suction pump device 110 provided as a contact auxiliary unit, the status of body tissue can be reliably observed from clear images without having to flush out the intestines and without being affected by body fluid or other foreign objects. In the case the patient has a disease such as an inflammatory bowel disease in particular, the status inside the intestines can be reliably observed while preventing exacerbation of symptoms caused by flushing out the intestines. In addition, since observations can be made along the entire length of the intestines, observations can be reliably made even at locations which were difficult to observe by conventional endoscopic examination, such as locations at a considerable distance from the anus.

In addition, in this in vivo observation device 101, driving of pump body 111 is preferably in the form of pulse driving. Namely, if body fluid is aspirated by continuously operating pump body 111, there is the possibility of a problem occurring with the capacity of battery 123 due to the suction force being too strong or power consumption becoming large. Consequently, the use of pulse driving allows the operation of pump body 111 to be in the form of intermittent operation corresponding to pulses, thereby facilitating adjustment of suction force and energy-saving operation.

Furthermore, in the present embodiment, although judgment section 122 judges whether or not in vivo observation device 101 has reached the intestines based on images captured with observation system 103, and suction pump device 110 is operated according to said judgment, the present embodiment is not limited to this, but rather a constitution may be employed in which, for example, the location in the body of in vivo observation device 101 is confirmed from outside the body, a signal is sent when the device has reached a desired location, and suction pump device 110 is operated when said signal has been received. Moreover, at this time, control section 120 may also be set so as to operate observation system 103 when said signal has been received. Whereupon, electrical power can be conserved since observation system 103 is only operated at the location that is desired to be observed.

### <Thirteenth Embodiment>

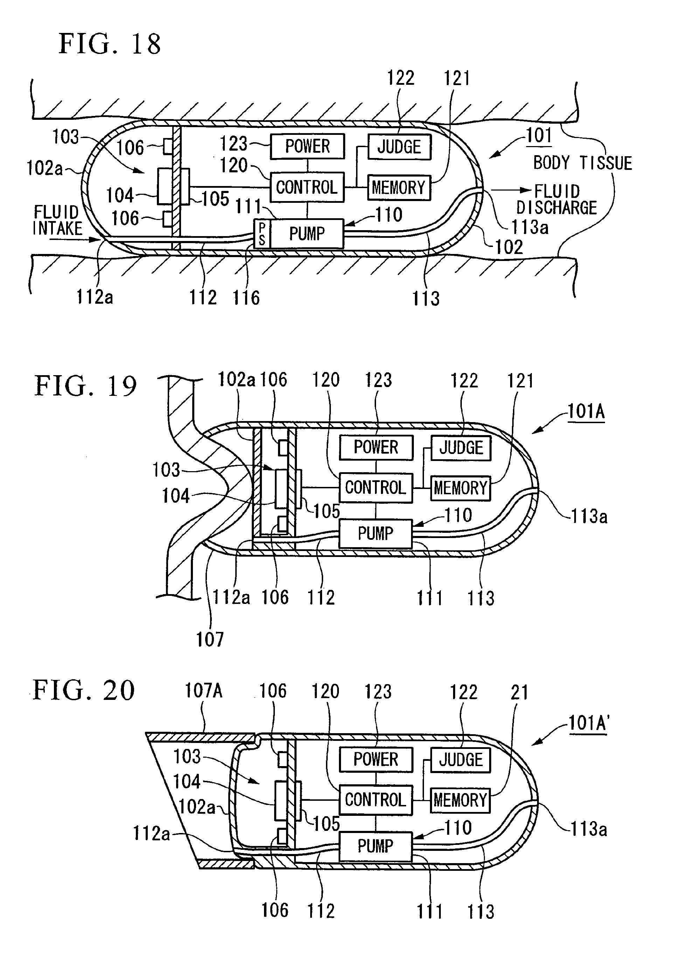

Next, an explanation is provided of a thirteenth embodiment of an in vivo observation device as claimed in the present invention with reference to Figs. 19 and 20. Furthermore, the same reference symbols are used to indicate those constituent features in the thirteenth embodiment that are the same as those in the twelfth embodiment, and their explanations are omitted.

In the thirteenth embodiment shown in Fig. 19, casing 102 of in vivo observation device 101A is provided with a cylindrical member in the form of a hood 107 that protrudes from the periphery of observation wall surface 102a towards the direction of observation. This hood 107 makes it possible to aspirate body fluid from opening 112a in observation wall surface 102a by opening up the end in the direction of observation.

As a result of providing this type of hood 107, in the case the end in the direction of observation of in vivo observation device 101A has approached body tissue to be observed to a certain degree, since the suctioned region is limited to a certain degree by hood 107, the body tissue can be efficiently and reliably suctioned toward in vivo observation device 101A.

In addition, a hood 107A may also be employed instead of the aforementioned hood 107 in which a cylindrical member is removably attached to the end of casing 102 which protrudes from the periphery of observation wall surface 102a towards the direction of observation as in, for example, in vivo observation device 101A' shown in Fig. 20. By employing this type of constitution, a hood 107A of a shape that is optimal for the application of in vivo observation device 110A' , namely for the shape or location and so forth of the body tissue to be observed, can be suitably selected and replaced. Furthermore, removable hood 107A shown in Fig. 20 has a shape in which the end opening is inclined with the side of opening 112a being shorter.

In addition, by producing the entirety or only the vicinity near the end of the aforementioned hood 107 or 107A from a flexible material, since the imparting of detrimental effects on the body tissue that is contacted during suctioning is improved, observations can be made without excessively suctioning the body tissue.

### <Fourteenth Embodiment>

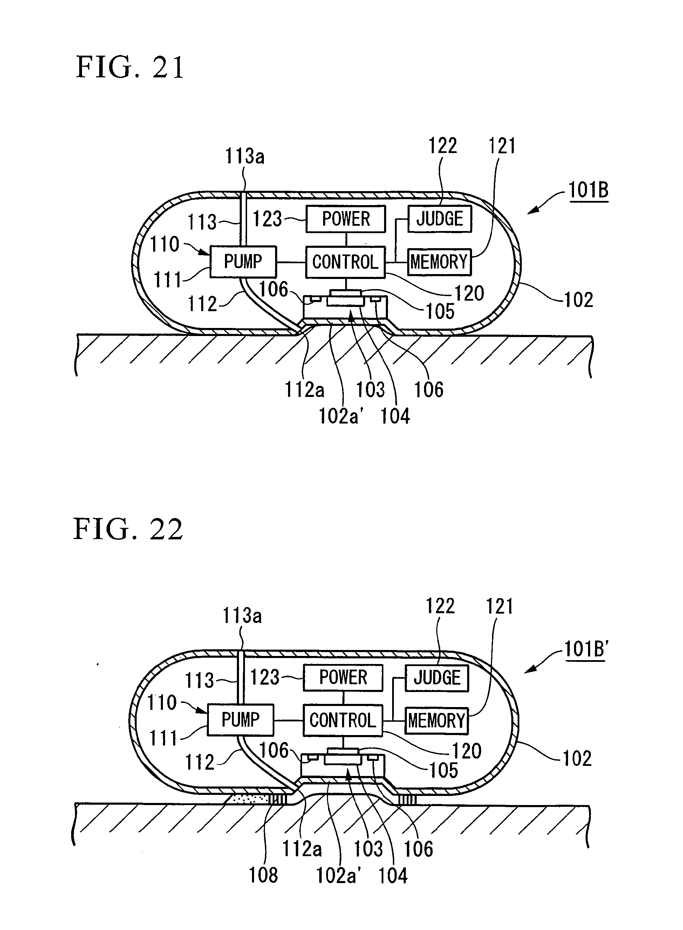

Continuing, an explanation is provided of a fourteenth embodiment of an in vivo observation device as claimed in the present invention with reference to Figs. 21 and 22.
Furthermore, the same reference symbols are used to indicate those constituent features in the fourteenth embodiment that are the same as those in the twelfth embodiment, and their explanations are omitted.

In the fourteenth embodiment shown in Fig. 21, in vivo observation device 101B is composed to be provided with an observation wall surface 102a' formed on one side of casing 102 having a capsular shape. This observation wall surface 102a' is provided with an indentation formed in the side of casing 102. An opening 112a of a duct 112 connected to suction pump device 110 is arranged in this observation wall surface 102a', and body fluid aspirated through opening 112a by the operation of pump body 111 is discharged from an opening 113a by passing through ducts 112 and 113 extending in the direction of diameter of casing 102 having a roughly circular cross-section.

According to this constitution, since capsular in vivo observation device 101B having a large width is engaged with body tissue on the outer periphery in a stable state, body tissue suctioned towards opening 112a along with body fluid can be made to closely adhere to observation wall surface 102a' by the operation of suction pump device 110. Thus, in the case in vivo observation device 101B observes a tubular organ like the intestines, and particularly in the case of observing body tissue having a horizontal section or nearly horizontal section, since in vivo observation device 101B can be easily maintained in a stable observation position, reliable observations can be made and clear captured images can be obtained.

In addition, in a in vivo observation device provided with an observation wall surface 102a' in its side as described above, a foreign object removal unit in the form of a brush 108 is preferably provided so as to surround the periphery of observation wall surface 102a' in the form of an indentation in the manner of, for example, in vivo observation device 101B' shown in Fig. 22. This brush 108 is provided in a state in which it protrudes from the outer periphery (side) of casing 102 at a suitable density, and not only removes foreign objects in the front with respect to the direction of travel during movement of in vivo observation device 101B', but also prevents foreign objects from entering the observation region from which body fluid and so forth has been removed through opening 112a. Thus, together with reducing the burden on suction pump device 110, a foreign object removal unit in the manner of brush 108 also is effective in maintaining a satisfactory observation environment.

### <Fifteenth Embodiment>

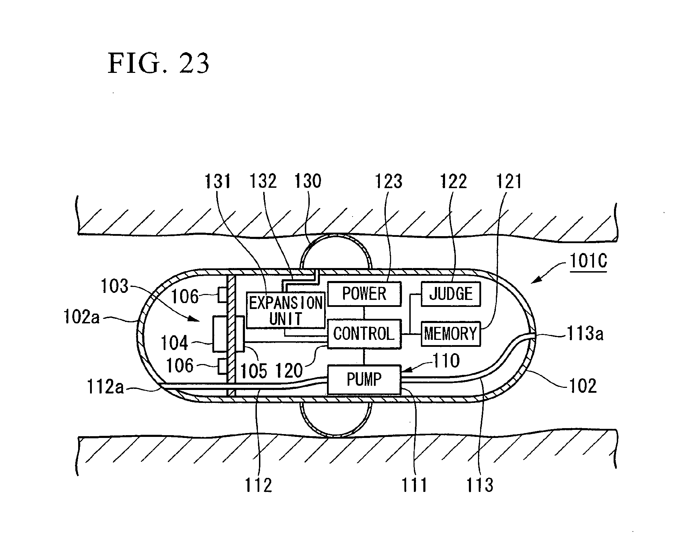

Continuing, an explanation is provided of a fifteenth embodiment of an in vivo observation device as claimed in the present invention with reference to Fig. 23. Furthermore, the same reference symbols are used to indicate those constituent features in the fifteenth embodiment that are the same as those in the twelfth through fourteenth embodiments, and their explanations are omitted.

In vivo observation device 101C of the present embodiment is provided with an outer diameter expanding unit in the form of balloon 130 on the side of casing 102. This balloon 130 is a ring-shaped member provided so as to surround the entire circumference of the side (outer periphery) of casing 102 to block large tubular organs in particular by expanding the outer diameter of in vivo observation device 101C when it expands.

Balloon 130 is expanded by supplying a compressed gas or other fluid through duct a 132 from an expansion unit 131 installed inside casing 102. Here, expansion unit 131 may be provided with a switching valve on a tank or other container that stores the compressed gas, or balloon 130 may be expanded by feeding body liquid aspirated with suction pump device 110 into balloon 130.

This expansion of balloon 130 is carried out by expansion unit 131 or suction pump device 110 being operated after receiving a signal output from control section 120 or a signal from outside the body under predetermined conditions in the same manner as suction pump device 110. When balloon 130 is expanded and closely adheres to the lumen, since the inside of the lumen is demarcated with balloon 130 serving as the boundary, body liquid can be efficiently aspirated by suction pump device 110.

### <Sixteenth Embodiment>

In the embodiments explained thus far, although a contact auxiliary unit has been provided inside an in vivo observation device (casing) in a state in which an observation wall surface closely contacted or adhered to body tissue during observation, in the embodiment explained below, an in vivo observation device closely contacted or adhered to body tissue by utilizing its own weight. Namely, the contact auxiliary unit that closely contacts or adheres the observation wall surface and body tissue during observation is in the form of a difference in specific gravity between body fluid and the in vivo observation device.

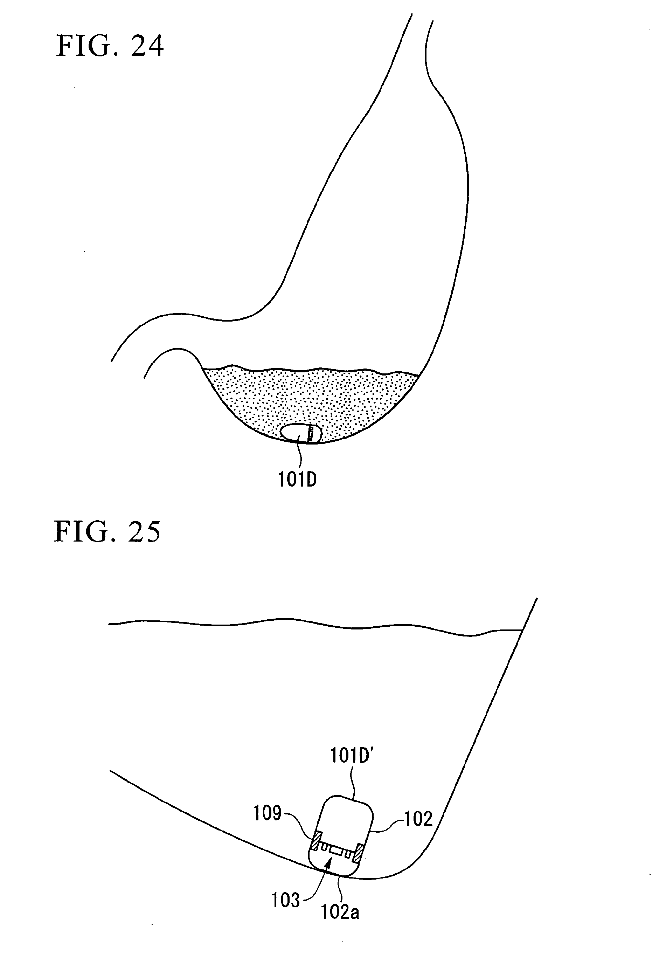

Figs. 24 and 25 show a in vivo observation device 101D that observes the inside of a large organ such as the stomach. This in vivo observation device 101D is set to have a specific gravity that is greater than the specific gravity of body fluid present in the body. Namely, in the example shown in the drawings, since in vivo observation device 101D, for which the specific gravity of the entire capsule is set to be larger than gastric juices, reliably sinks in the gastric juices due to its own weight, observations can be made without gastric juices obstructing the field of view in a state in which it is closely adhered to the body tissue of the stomach wall. In this case, by suitably changing the body position of the patient who swallowed in vivo observation device 101D, since in vivo observation device 101D moves to a lower location due to its own weight, body tissue can be thoroughly observed even in the case of large organs like the stomach.

Furthermore, in vivo observation device 101D in this case may be used after changing the specific gravity setting of each of the aforementioned in vivo observation devices, or suction pump device 110 may be removed.

The center of gravity of the aforementioned in vivo observation device 101D is preferably shifted to the side of observation wall surface 102a.

As shown in Fig. 25, a in vivo observation device 101D' having its center of gravity on the side of observation wall surface 102a should be adjusted so that the center of gravity shifts to the side of observation wall surface 102a by providing a weight 109, for example, or the location of the center of gravity should be adjusted by making contrivances in the arrangement of each constituent part housed within casing 102. As a result of employing this type of constitution, since observation wall surface 102a, which faces downward due to gravity when the device sinks in body fluid, closely adheres to body tissue while facing that body tissue, observations can be made more reliably.

### <Seventeenth Embodiment>

Continuing, an explanation is provided of the case in which a contact auxiliary means which closely contacts the observation wall surface to body tissue during observation uses pressure as the action from outside the body.

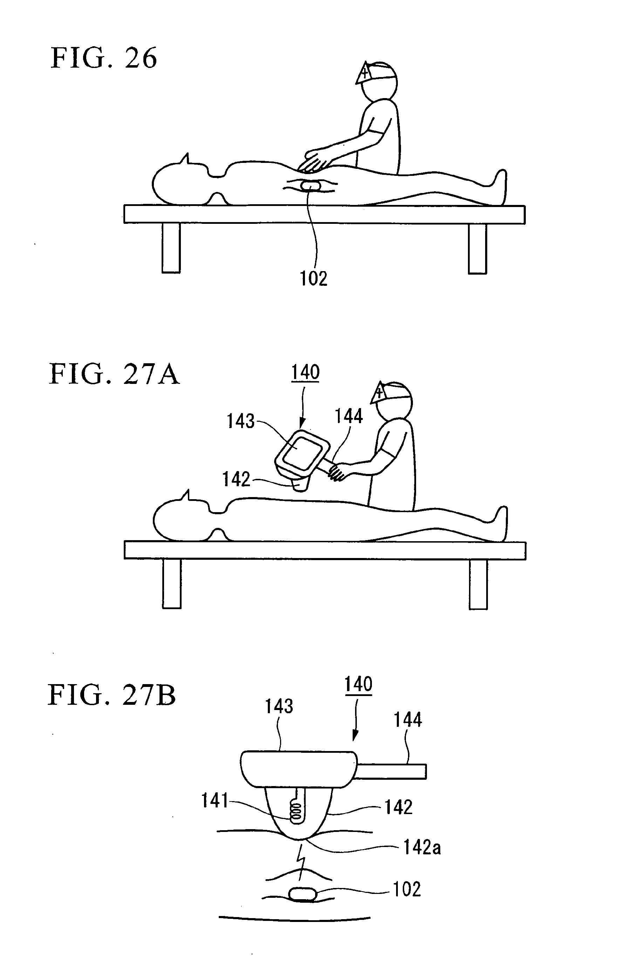

In the example shown in Fig. 26, once in vivo observation device 101 that has been taken orally into the body has reached a desired location to be observed, pressure is applied from outside the body near the organ to be observed to closely adhere the body tissue to observation wall surface 102a. Since this type of procedure is carried out by a physician applying pressure manually while viewing an image, special tools are not required and the body tissue can be observed easily. Furthermore, the images in this case may be those sent from in vivo observation device 101 or those acquired from outside the body.

In the example shown in Figs. 27A and 27B, an oppressor of an external contact auxiliary device provided with an operation generating section that generates an operation in casing 2 from outside the body is used as a contact auxiliary unit instead of the hands of a physician. This oppressor 140 is composed by being equipped with a pressing section 142 in which is provided an antenna (receiving unit) that receives data transmitted from a transmission unit (not shown) provided within casing 102, a display section 143 that displays the received data on a screen, and a grip 144 for performing pressing with this device.

Antenna 141 detects image data transmitted from casing 102 located within the body, signals for detecting location and so forth, and in the example of the constitution shown in the drawings, is contained within protruding pressing section 142 of which the pressing end 142a is curved. Image data and location detection signals detected with this antenna 141 are displayed on a display section 143 such as a liquid crystal monitor, EL monitor or plasma monitor.

The aforementioned oppressor 140 is used to perform pressing by, for example, a physician pressing on a patient's body while viewing images on display section 143 by holding onto grip 144 for performing pressing. The following provides a brief explanation of that procedure.

After waiting for a suitable amount of time after the patient has been orally administered casing 102, when a oppressor 140 that has been turned on with a switch not shown is brought close to the patient's body, image data transmitted from casing 102 and location information of casing 102 are displayed on display section 143. After confirming that casing 102 is near the target observation location based on this location information, the physician operates oppressor 140 while viewing the image data to judge the direction, intensity and so forth by which end 142a of oppressor 142 is pressing. Casing 102 and the body tissue to be observed are then closely adhered for observation by applying suitable pressure with oppressor 142.

By performing the procedure using oppressor 140 in this manner, acquisition of information and the pressing procedure can be carried out with a single device as well as within the same field of view, thereby reducing the burden on the physician by improving the ease of the procedure.

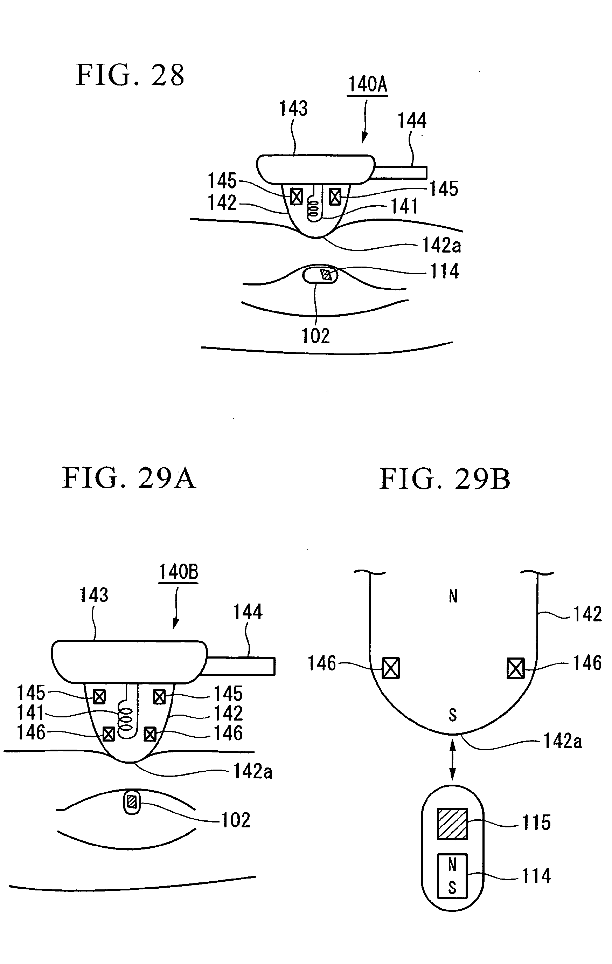

Next, an explanation is provided of a first variation of the aforementioned oppressor 140 as shown in Fig. 28. Furthermore, the same reference symbols are used to indicate those constituent features that are the same as those of the aforementioned oppressor 140, and their explanations are omitted.

Oppressor 140A of the first variation differs in that an electromagnet (magnetic coil) 145 capable of suitably being switched on and off is provided within pressing section 142. In addition, a magnet 114 that is attracted by the magnetic attraction from oppressor 140A is required for casing 102 used as a pair with this type of oppressor 140A. Although this magnet 114 may be provided by adding exclusively for this purpose, an internal part made of metal and so forth that can be magnetically attracted by electromagnet 145 may also be used. Namely, electromagnet 145 in this case functions as a magnetic field generating device of the operation generating section, while magnet 114 functions as the operated section. Furthermore, a ferromagnetic body can also be used instead of magnet 114.

As a result of employing this type of constitution, if the switch not shown for an electromagnetic provided at a suitable location on oppressor 140A is switched on at a location to be observed, current is supplied to electromagnet 145 resulting in the generation of magnetic attractive force. Consequently, casing 102 at the observed location in the body magnetically attracts magnet 114 causing it to be attracted towards oppressor 140A. As a result, since the in vivo observation device is closely adhered to the wall surface inside a lumen, casing 102 and body tissue to be observed are closely adhered enabling observation of that body tissue, thereby further improving the ease of operation during observation.

Continuing, an explanation is provided of a second variation of the aforementioned oppressor 140. Furthermore, the same reference symbols are used to indicate those constituent features that are the same as those of the aforementioned oppressors 140 and 140A, and their explanations are omitted.

Oppressor 140B of this second variation differs in that it is provided with an antenna 146 for supplying electrical power in addition to oppressor 140A of the aforementioned first variation. In addition, an antenna for receiving electrical power 115 that generates electricity in collaboration with antenna 146 for supplying electrical power is required for casing 102 used as a pair with this type of oppressor 140B.

As a result of employing this type of constitution, when electromagnetic attractive force is generated by supplying power to electromagnet 145 at a location to be observed, magnet 114 of casing 102 is attracted towards oppressor 140B. At this time, if magnet 114 is a permanent magnet having a magnetic poles, then antenna 146 for supplying electrical power and antenna 115 for receiving electrical power are directly opposed according to the orientation of the magnetic poles on the side of electromagnet 145. Namely, as shown in Fig. 29B, in the case the magnetic poles on the side of pressing section 142 formed by electromagnet 145 are taken to be the S pole on the side of end 142a and the N poles on the side of display section 143, then the N pole of magnet 114 is attracted to the S pole of electromagnet 145 in the in vivo observation device.

Thus, if observation wall surface 102a is arranged on the side of the N pole of in vivo observation device 101, since observation wall surface 102a is directly opposed to the inner wall surface of the lumen as a result of being subjected to the magnetic attractive force from outside the body, body tissue of the lumen can be reliably observed.

In addition, if antenna 146 for supplying electrical power and antenna 115 for receiving electrical power are in a positional relationship such that they are directly opposed, they are in a state in which power is generated with the greatest efficiency, and since electrical power is supplied to the battery within the casing, in vivo observation device 101 is able to withstand use for a long period of time.

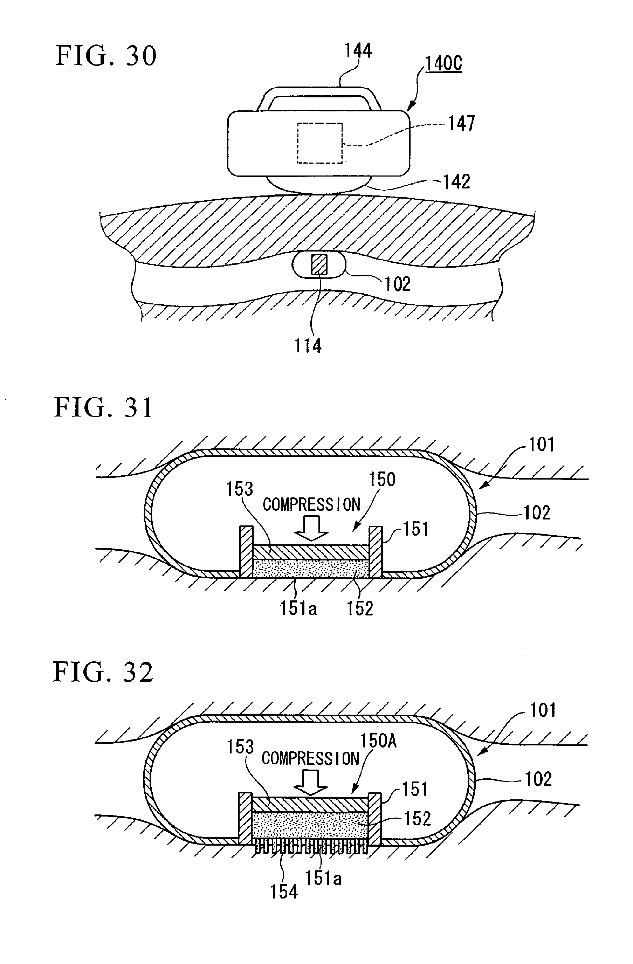

Continuing, an explanation is provided of a third variation of the aforementioned oppressor 140 as shown in Fig. 30. Furthermore, the same reference symbols are used to indicate those constituent features that are the same as those of the aforementioned oppressors 140, 140A and 140B, and their explanations are omitted.

In this third variation, a magnetic sensor 147 is provided within oppressor 140C that serves as a location detection unit of casing 102. This magnetic sensor 147 is able to accurately detect the location of casing 102 by detecting a magnet 114 installed inside casing 102.

Even if this constitution is employed, since the current location of casing 102 in the body can be accurately recognized using a comparatively simple constitution, observations can be made by applying pushing pressure at a suitable location from outside the body to closely adhere casing 102 to the wall surface inside a lumen.

### <Eighteenth Embodiment>

However, although each of the aforementioned embodiments has a contact auxiliary unit that closely contacts or adheres casing 102 to body tissue desired to be observed, and obtained satisfactory observation images by preventing obstruction of the field of view by body fluid and so forth, in the following embodiment, an explanation is provided by indicating that in which the aforementioned casing 102 is provided with a drug administration unit that accurately administers a drug by closely contacting or adhering to a target body tissue.
Furthermore, the drawings used in the following explanation primarily show only the drug administration unit, while the observation system, contact auxiliary unit and control section, etc. of each of the aforementioned embodiments are omitted.

A drug administration unit 150 shown in Fig. 31 is composed by installing a sponge-like drug storage section 152 impregnated with a drug within a cylinder chamber 151 provided with drug administration opening 151a that opens to the outer periphery of casing 102 so as to administer a drug by pressing out the drug by compressing this drug storage section 152 with a piston 153 from the inside of casing 102. The drug storage section 152 in this case functions as a drug storage/transport unit that stores and transports the drug within casing 102.

A drug is administered by this drug administration unit 150 by first confirming that the body tissue closely contacted or adhered to by the aforementioned observation system 103 is the body tissue to which the drug is to be administered, and then operating piston 153 according to, for example, a drug administration signal from control section 120 or a drug administration signal from outside the body. Namely, when pressure is applied by pushing piston 153 into cylinder chamber 151 in the state in which drug storage section 152 is closely contacted to the body tissue at an affected area to which drug is to be administered, the drug stored so as to be impregnated in drug storage section 152 is pushed out from drug administration opening 151a, thereby enabling the drug to be directly and accurately applied to the body tissue of the affected area.

Next, an explanation is provided of a first variation of the aforementioned drug administration unit 150 as indicated in Fig. 32. Furthermore, the same reference symbols are used to indicate those constituent features that are the same as those of the aforementioned drug administration unit 150 shown in Fig. 30, and their explanations are omitted.

Drug administration unit 150A of the first variation has a plurality of micro-needles 154 provided in drug administration opening 151a of drug storage section 152 functioning as a drug storage/transport member. Since these needles 154 are inserted into body tissue as a result of being closely contacted, if pressure is applied by pushing in piston 153 from this state, the drug within drug storage section 152 is pushed out and is directly injected into the body tissue at an affected area from needles 154. Thus, since the drug can be accurately injected and administered to body tissue of an affected area, the drug can be administered efficiently.

However, the drug storage section 152 in this case is naturally that which is able to be employed in the manner of a sponge as previously described. However, the present variation is not limited to this, but rather, for example, a plurality of needles 154 may also be provided on a plate-shaped member fastened to cylinder chamber 151 so that drug administration opening 151a is obstructed by withstanding the pressure of piston 153.

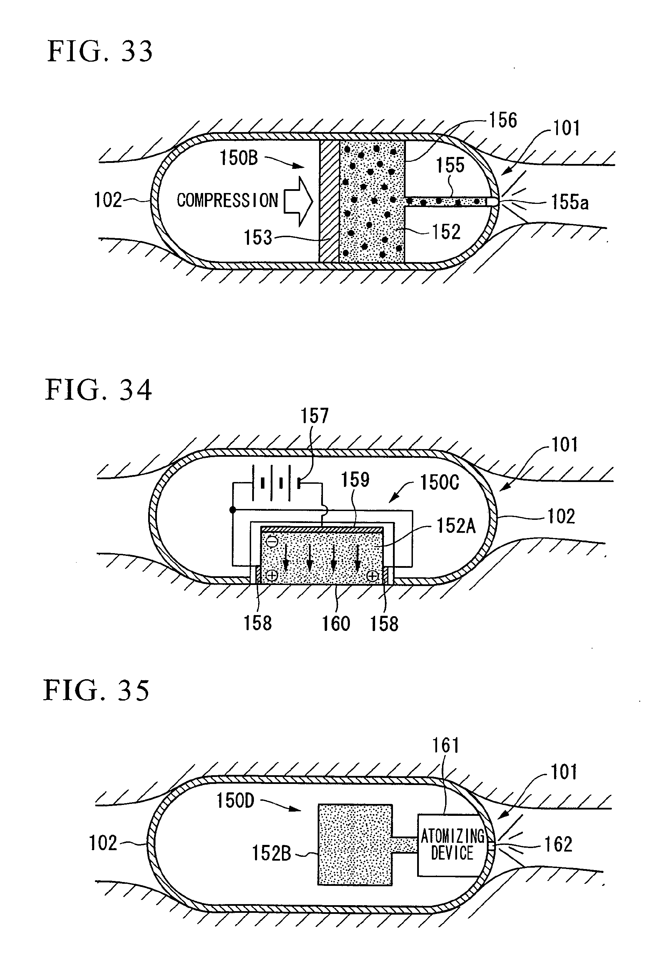

Continuing, an explanation is provided of a second variation of the aforementioned drug administration unit 150 as indicated in Fig. 33. Furthermore, the same reference symbols are used to indicate those constituent features that are the same as those of the aforementioned drug administration unit and its variation, and their explanations are omitted.

In drug administration unit 150B of the second variation, a gel-like substance impregnated with a drug is stored in drug storage section 152 of a drug storage/transport unit. Since this substance is pushed out and released near an affected area by operating piston 153 near a target of drug administration, a gel-like substance containing a drug is affixed to body tissue of the affected area. As a result, since the gel-like substance remains at the affected area over a long period of time, the drug is gradually released from this substance, enabling the drug to be administered over a long period of time.

In addition, in the example shown in the drawing, a cylinder chamber is formed by using the outer peripheral wall of casing 102, and the space where this cylinder chamber is divided with wall surface 156 provided with drug administration duct 155 serves as drug storage section 152 for storage of drug.
Furthermore, since drug administration duct 155 forms a communicating state between drug storage section 152 and the outside of casing 102, and the end opens in the wall surface of casing 102 to form drug administration port 155a, drug that is pushed out as a result of being compressed by the operation of piston 153 is released near an affected area from drug administration port 155a.

Continuing, an explanation is provided of a third variation of the aforementioned drug administration unit 150 as indicated in Fig. 34. Furthermore, the same reference symbols are used to indicate those constituent features that are the same as those of the aforementioned drug administration unit and its variations, and their explanations are omitted.

In drug administration unit 150C of the third variation, a gel-like drug stored in drug storage section 152A is led to the surface of casing 102 by electrophoresis where it is directly applied to closely contacted body tissue. In this case, electrodes in the form of positive electrode 158 and negative electrode 159 connected to a power source 157 are arranged in drug storage section 152A.

Positive electrode 158 is arranged on the side of the drug administration surface serving as the outer periphery of drug storage section 152A, namely the side of drug administration opening 160 that opens in casing 102. In addition, negative electrode 159 is arranged on the bottom of drug storage section 152A that serves as the central side of casing 102. Thus, when power is supplied as a result of power source 157 being switched on after casing 102 has reached the site of a predetermined affected area, since electrophoresis is generated in positive electrode 158 and negative electrode 159, the gel-like drug is slowly led to drug administration opening 160, and is directly applied to body tissue that closely contacts to casing 102. Consequently, drug within drug storage section 152A can be reliably applied to body tissue of an affected area over a long period of time and over a wide range.

Continuing, an explanation is provided of a fourth variation of the aforementioned drug administration unit 150 as indicated in Fig. 35. Furthermore, the same reference symbols are used to indicate those constituent features that are the same as those of the aforementioned drug administration unit and its variations, and their explanations are omitted.

In drug administration unit 150D of the fourth variation, a drug stored in drug storage section 152B of a container provided as a drug storage/transport unit is released after atomizing with atomizing device 161. If a constitution is employed that is provided with this type of atomizing device 161, a drug can be dispersed and administered over a wide range of body tissue. Furthermore, reference symbol 162 in the drawing indicates a release port opened in casing 102.

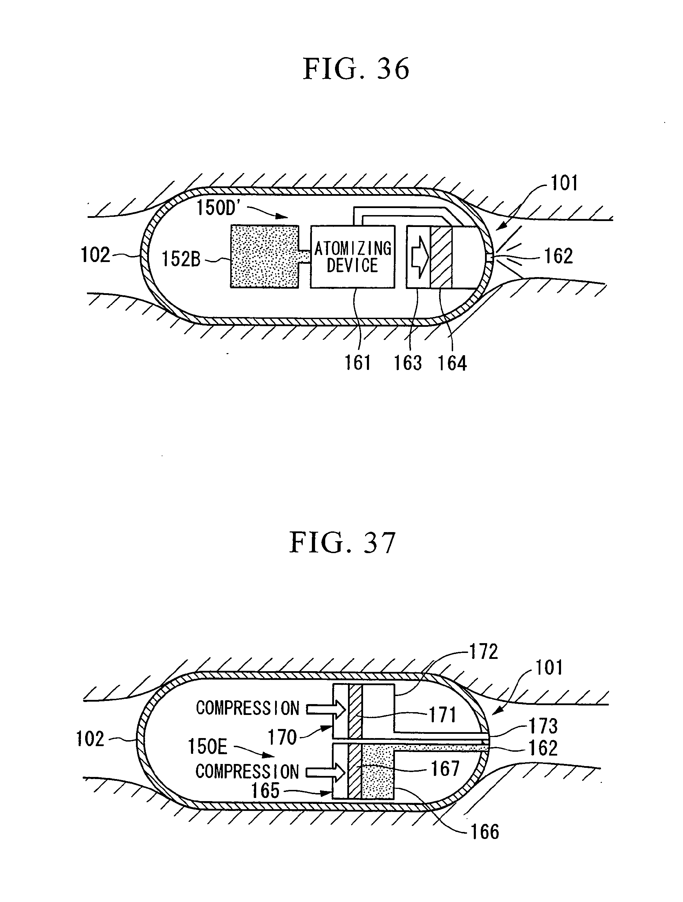

In addition, a drug administration unit 150D of the aforementioned fourth variation preferably pushes out atomized drug by compressing the atomized drug in the manner of drug administration unit 150D' of a fifth variation shown in Fig. 36. Namely, as a result of pressurizing and releasing a drug atomized with atomizing device 161 with a piston 164 that slides within a cylinder 163, as compared with the case of releasing the drug simply by atomizing, the drug can be administered over an even wider range. This type of pressurized release is particularly suitable for drug administration to long organs such as the intestines.

Continuing, an explanation is provided of a sixth variation of the aforementioned drug administration unit 150 as indicated in Fig. 37. Furthermore, the same reference symbols are used to indicate those constituent features that are the same as those of the aforementioned drug administration unit and its variations, and their explanations are omitted.

In drug administration unit 150E of the sixth variation, compressed air releasing device 170 is provided as a foreign object removal unit that removes body fluid and so forth present on the surface of an affected area by releasing air and so forth prior to drug administration. This compressed air releasing device 170 sprays air compressed by a piston 171 within a cylinder 172 at the vicinity of an affected area from a release port 173, and body fluid and other foreign objects adhered to the surface of body tissue can be removed by the spraying force of this air. Thus, body tissue at an affected area exposed with observation system 103 of casing 102 can be confirmed and drug can be reliably administered. Furthermore, this type of foreign object removal unit can also be used to secure a satisfactory field of view for the aforementioned observation system 103 by removing foreign objects near the affected area.

In addition, in the case of being provided with the aforementioned foreign object removal unit, procedures are carried out in the order of foreign object removal followed by drug administration. In the constitution example shown in Fig. 37, a drug releasing device 165 is provided that is composed in the same manner as the aforementioned compressed air releasing device 170. In this case, after foreign objects have been removed with compressed air releasing device 170, drug releasing device 165 is operated and drug stored in cylinder 165 is compressed by piston 167. Thus, the drug inside cylinder 166 which also functions as a drug storage section is subjected to compression by piston 167 and released to the outside of casing 102 from release port 162, thereby enabling the drug to be reliably administered to body tissue from which foreign objects have been removed.

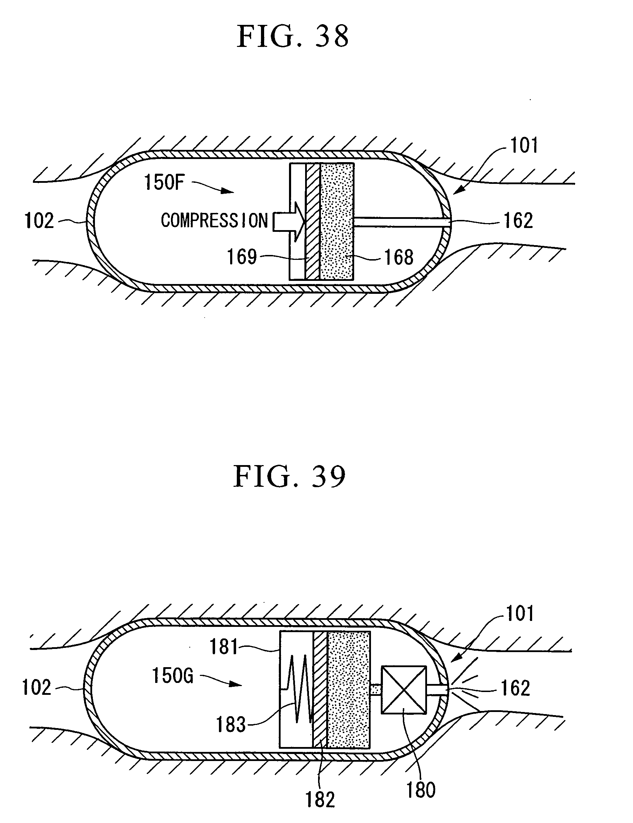

Continuing, an explanation is provided of a seventh variation of the aforementioned drug administration unit 150 as indicated in Fig. 38. Furthermore, the same reference symbols are used to indicate those constituent features that are the same as those of the aforementioned drug administration unit and its variations, and their explanations are omitted.

In drug administration unit 150F of the seventh variation, a constitution is employed in which a drug is administered by automatically performing foreign object removal and drug administration in that order. This drug administration unit 150F is equipped with a cylinder 168 and a piston 169, and is composed such that air and drug stored in cylinder 168 are pushed out with piston 169. Namely, as a result of storing the drug on the side of piston 169 in cylinder 168, and storing air on the side of release port 162 that opens in casing 102, after initially releasing air from release port 162, drug can be subsequently released automatically. Thus, a drug can be automatically administered directly to body tissue targeted for drug administration after first removing foreign objects present on the surface of the body tissue.

Continuing, an explanation is provided of an eighth variation of the aforementioned drug administration unit 150 as indicated in Fig. 39. Furthermore, the same reference symbols are used to indicate those constituent features that are the same as those of the aforementioned drug administration unit and its variations, and their explanations are omitted.

Drug administration unit 150G of the eighth variation is characterized by the use of a magnetic response valve 180 that is opened and closed by the approach of a magnet from outside the body. This magnetic response valve 180 is arranged in a drug outlet flow path that communicates with a release port 162 from the outlet of a cylinder 181 in which a drug is stored. Furthermore, a piston 182 inside cylinder 181 is subjected to force in the direction in which drug is pushed out by a spring 183.

Drug administration unit 150G composed in this manner stores a drug within cylinder 181 in the state in which magnetic response valve 180 is closed. Thus, once casing 102 has been confirmed to have reached an affected site targeted for drug administration, a magnet approaches the vicinity of the affected area from outside the body and magnetic response valve 180 opens. As a result, since piston 182 subjected to the force of spring 183 pushes out the drug, drug is administered to body tissue from release port 162.

As has been explained above, an in vivo observation device of the present invention is provided with a contact auxiliary unit that removes body fluid and other foreign objects by causing the observation wall surface to closely contact or adhere to body tissue to be observed in order to secure a satisfactory field of view for observation system 103.

In addition, as a result of the in vivo observation device being provided with a drug administration unit capable of administering a drug in a state in which the device is closely adhered to body tissue, the drug can be accurately and reliably administered to a target affected area.

Furthermore, the present invention is not limited to the aforementioned embodiments, but rather can be suitably modified within a range that does not deviate from the scope or spirit of the present invention.

### INDUSTRIAL APPLICABILITY

According to the in vivo observation device of the present invention, since body tissue can be observed by expanding a balloon causing it to closely adhere to body tissue and then observing body tissue through said balloon, the status of the body tissue can be reliably observed. In the case of inflammatory bowel diseases in particular, the status inside the digestive tract, including any affected areas, can be reliably observed while preventing exacerbation of symptoms caused by flushing the intestines.

In addition, according to the in vivo observation device of the present invention, since body tissue can be observed in a state in which an observation wall surface and body tissue are closely adhered by a contact auxiliary device, the status of body tissue can be reliably observed without having to flush the intestines and without the field of view being obstructed by body fluid, gas and other fluids. In the case of inflammatory bowel diseases in particular, the status of the digestive tract, including any affected areas, can be reliably observed while preventing exacerbation of symptoms caused by flushing the intestines.

## Claims

1. An in vivo observation device comprising:
a capsular casing that is taken orally into a living body;
an observation device provided within said casing which observes the inside of the living body through an observation wall surface of said casing; and
a contact auxiliary device that causes the observation wall surface to closely contact to body tissue during observation.

2. An in vivo observation device according to claim 1, wherein the contact auxiliary device is provided within the casing.

3. An in vivo observation device according to claim 1, wherein the contact auxiliary device is an external contact auxiliary device provided with an action generating section that generates an action in the casing from outside the living body.

4. An in vivo observation device according to claim 1, wherein the contact auxiliary device comprises an external contact auxiliary device provided with an action generating section that generates an action in the casing from outside the living body, and an reacting section that receives the action generated by the action generating section provided in the casing.

5. An in vivo observation device according to claim 2, wherein the contact auxiliary device has a fluid transfer device installed within the casing which aspirates fluids such as body fluids and gases within the living body and causes them to flow from the front of the observation wall surface to the rear, and causes the body tissue to be closely contacted to the observation wall surface as the fluids are aspirated by the fluid transfer device.

6. An in vivo observation device according to claim 5, wherein the casing is provided with a cylindrical member that protrudes from the observation wall surface towards the direction of observation.

7. An in vivo observation device according to claim 6, wherein the cylindrical member is removable.

8. An in vivo observation device according to claim 5, wherein the observation wall surface is provided on the side portion of the casing.

9. An in vivo observation device according to claim 8, wherein a foreign object removal device that protrudes from the outer periphery of the casing is provided around the observation wall surface.

10. An in vivo observation device according to claim 5, wherein an outer diameter expansion device is provided on the side of the casing.

11. An in vivo observation device according to claim 2, further comprising:
a capsular casing that is taken orally into the living body;
an observation device provided within the casing which observes the inside of the living body;
an optically transparent balloon provided on the casing so as to cover the periphery of the observation device and which can be expanded so as to closely contact to the living body when moving inside the living body; and
an expansion device that expands the balloon by supplying a fluid inside the balloon,
wherein the observation device observes the inside of the living body through the balloon.

12. An in vivo observation device according to claim 11, wherein the expansion device is provided with an acquisition section that acquires body fluid inside the living body, and an expansion section that expands the balloon based on the liquid content of the body fluid acquired with the acquisition section.

13. An in vivo observation device according to claim 11,
wherein:
a duct that connects the outside and inside of the balloon is provided in the casing; and
the expansion device is provided with a pump that expands the balloon by supplying fluid from the outside to the inside of the balloon through the duct or contacts the balloon by discharging fluid to the outside from inside the balloon, and a control section that controls the operation of the pump.

14. An in vivo observation device according to claim 11,
wherein:
another balloon is provided in the casing;
ducts that respectively communicate with the insides of the balloon and the other balloon are provided within the casing; and
the expansion device is provided with a pump that mutually supplies the fluid to both balloons through the ducts, and a control section that controls the operation of the pump.

15. An in vivo observation device according to claim 11, wherein the balloon is provided with a reversing prevention device that has projections on the outer surface which protrude towards the rear with respect to the direction of travel.

16. An in vivo observation device according to claim 11,
wherein:
the balloon has an electrode on its outer surface; and
an electrical power supply section is provided within the casing that supplies electrical power to the electrode.

17. An in vivo observation device according to claim 11,
wherein:
the fluid is an optically transparent liquid drug;
micropores are provided in the balloon that allow the drug to be discharged outside the balloon when the balloon has been expanded at a pressure equal to or greater than a predetermined value; and
the expansion device has a pressure raising device that raises the pressure inside the balloon to the pressure equal to or greater than the predetermined value.

18. An in vivo observation device according to claim 11,
wherein:
the fluid is an optically transparent liquid drug; and
a duct that connects the outside and inside of the balloon, a switching valve that can open and close the duct and release drug inside the balloon to the outside, and a switching valve control section that controls operation of the switching valve, are provided in the casing.

19. An in vivo observation device according to claim 11, wherein a drug storage section that stores a drug, a duct that connects the drug storage section with the outside of the balloon, a releasing device interposed in the duct which releases drug stored in the drug storage section outside the balloon, and a control section that operates the releasing device, are provided in the casing.

20. An in vivo observation device according to claim 14,
wherein:
a releasing device is provided within the balloon that releases the fluid outside the balloon when pressure within the balloon has reached a pressure equal to or greater than a predetermined value;
the fluid is an optically transparent drug; and
the control section operates the pump so that the pressure within the balloon reaches a pressure equal to or greater than a predetermined value.

21. An in vivo observation device according to claim 17,
wherein:
another balloon is provided in the casing that has an electrode on its external surface and stores the drug inside;
an electrical power supply section that supplies electrical power to the electrode and ducts that respectively communicate with the insides of the first balloon and the other balloon are provided within the casing; and
the electrical power supply section supplies electrical power to the electrode to release the drug.

22. An in vivo observation device according to claim 11, wherein the refractive index of the balloon is equal to or lower than the refractive index of the fluid.

23. An in vivo observation device according to claim 2, wherein the contact auxiliary device is the difference in specific gravity of the entire device such that the specific gravity of the entire device is set to be larger than the specific gravity of the fluid present in the living body.

24. An in vivo observation device according to claim 23, wherein the center of gravity is decentered towards the observation wall surface.

25. An in vivo observation device according to claim 3, wherein:
the action generating section is a pressing section that presses against the living body; and
a grip for operating pressing is provided in the external contact auxiliary device.

26. An in vivo observation device according to claim 25,
wherein:
a transmission device that transmits data is provided within the casing; and
a display section that displays data transmitted from the transmission device is provided in the pressing section.

27. An in vivo observation device according to claim 25, wherein a location detecting device that detects the location of the casing is provided in the external contact auxiliary device.

28. An in vivo observation device according to claim 25,
wherein:
a permanent magnet for electromagnetic attraction and an antenna for receiving electrical power are provided within the casing; and
a coil for magnetic attraction and an antenna for supplying electrical power are provided in the pressing section.

29. An in vivo observation device according to claim 4,
wherein:
the action generating section is a magnetic field generating device; and
the reacting section is a permanent magnet or a ferromagnetic body.

30. An in vivo observation device according to claim 29,
wherein:
a transmission device that transmits data is provided within the casing; and
a display section that displays data transmitted from the transmission device is provided in the pressing section.

31. An in vivo observation device according to claim 29, wherein a location detecting device that detects the location of the casing is provided in the external contact auxiliary device.

32. An in vivo observation device according to claim 29,
wherein:
a permanent magnet for electromagnetic attraction and an antenna for receiving electrical power are provided within the casing; and
a coil for magnetic attraction and an antenna for supplying electrical power are provided in the pressing section.

33. An in vivo observation device according to claim 1, wherein a drug administration device is provided that administers a drug at a desired site from the casing when the casing is closely contacted to the body tissue.

34. An examination method comprising:
a step in which an in vivo observation device is introduced into a subject;
a step in which the location of the in vivo observation device within a living body is recognized;
a step in which the in vivo observation device is closely contacted to a body wall based on the recognized location; and
a step in which an image of the closely contacted section is acquired.

35. An examination method according to claim 34, wherein the step in which the location within the living body is recognized comprises recognizing with an image acquired by the in vivo observation device.

36. An examination method according to claim 34, wherein the step in which the location within the living body is recognized comprises recognizing according to a timer installed in the in vivo observation device.

37. An examination method according to claim 34, wherein the step in which the location within the living body is recognized comprises recognizing based on location information of the in vivo observation device.

38. An examination method according to claim 34, wherein the step in which the in vivo observation device is closely contacted to a body wall comprises closely contacting by a fluid transfer device that aspirates a fluid such as body fluid or gas in the living body from the front in the direction of observation of the in vivo observation device and causes it to flow out to the rear.

39. An examination method according to claim 34, wherein the step in which the in vivo observation device is closely contacted to a body wall comprises closely contacting a balloon to a body wall by supplying a fluid to an optically transparent balloon provided in the observation section in the in vivo observation device to expand the balloon.

40. An examination method according to claim 34, wherein the step in which the in vivo observation device is closely contacted to a body wall is a step in which pushing pressure is applied from outside the living body to a portion where the in vivo observation device is present.

41. An examination method according to claim 34, wherein the step in which the in vivo observation device is closely contacted to a body wall comprises closely contacting by generating a magnetic field outside the living body and attracting a magnet or ferromagnetic body installed within the in vivo observation device.

42. An examination and treatment method comprising:
a step in which an in vivo observation device is introduced into a subject;
a step in which the location within a living body is recognized;
a step in which the in vivo observation device is closely contacted to a body wall based on the recognized location;
a step in which an image of the closely contacted section is acquired;
a step in which the acquired image is confirmed; and
a step in which a drug is released if necessary.

43. An examination and treatment method according to claim 42,
wherein the step in which the location within the living body is recognized comprises recognizing with an image acquired by the in vivo observation device.

44. An examination and treatment method according to claim 42,
wherein the step in which the location within the living body is recognized comprises recognizing according to a timer installed in the in vivo observation device.

45. An examination and treatment method according to claim 42,
wherein the step in which the location within the living body is recognized comprises recognizing based on location information of the in vivo observation device.

46. An examination and treatment method according to claim 42,
wherein the step in which the in vivo observation device is closely contacted to a body wall comprises closely contacting by a fluid transfer device that aspirates a fluid such as body fluid or gas in the living body from the front in the direction of observation of the in vivo observation device and causes it to flow out to the rear.

47. An examination and treatment method according to claim 42,
wherein the step in which the in vivo observation device is closely contacted to a body wall comprises closely contacting a balloon to a body wall by supplying a fluid to an optically transparent balloon provided in the observation section in the in vivo observation device to expand the balloon.

48. An examination and treatment method according to claim 42,
wherein the step in which the in vivo observation device is closely contacted to a body wall is a step in which pushing pressure is applied to a portion where the in vivo observation device is present from outside the living body.

49. An examination and treatment method according to claim 42,
wherein the step in which the in vivo observation device is closely contacted to a body wall comprises closely contacting by generating a magnetic field outside the living body and attracting a magnet or ferromagnetic body installed within the in vivo observation device.
